(19) Europäisches Patentamt
European Patent Office
Office européen des brevets

(11) Publication number: 0 593 936 A1

# (12) EUROPEAN PATENT APPLICATION

(21) Application number: 93115443.9

(22) Date of filing: 24.09.93

(51) Int. Cl.5: C07D 249/20, C08K 5/3475, C07D 401/12, C07F 7/18, C08K 5/54

(30) Priority: 24.09.92 US 949947

(43) Date of publication of application: 27.04.94 Bulletin 94/17

(84) Designated Contracting States: AT BE CH DE DK ES FR GB IT LI LU NL SE

(71) Applicant: ELF ATOCHEM NORTH AMERICA, INC.
2000 Market Street, 26th Floor
Philadelphia Pennsylvania 19103-3222(US)

(72) Inventor: MacLeay, Ronald E.
10 Mahogany Drive
Williamsville, New York 14221(US)
Inventor: Kmiec, Jennifer P.
517 Montrose Avenue
Kenmore, New York 14223(US)
Inventor: Stein, Daryl L.
9667 Roundhouse Drive
West Chester, Ohio 45069(US)

(74) Representative: Abitz, Walter, Dr.-Ing. et al
Patentanwälte Abitz & Partner
Postfach 86 01 09
D-81628 München (DE)

## (54) N-[2-hydroxy-3-(2H-benzotriazol-2-yl)benzyl]oxamides as light stabilisers.

(57) This invention relates to novel UV absorbers, their methods of preparation, their use in stabilizing polymers and polymers so stabilized against the degradative effects of UV light. The novel compounds of formula I are oxamides which are substituted on one or both nitrogens with a 2-hydroxy-3-(2H-benzotriazol-2-yl)benzyl group. The 2-hydroxy-3-(2H-benzotriazol-2-yl)benzyl groups are efficient UV absorbing groups and the oxamide group contributes additional light stabilizing, heat stabilizing and metal complexing properties to the compounds. The compounds have low-volatility and are especially useful as UV stabilizers for engineering resins processed at high temperatures.

EP 0 593 936 A1

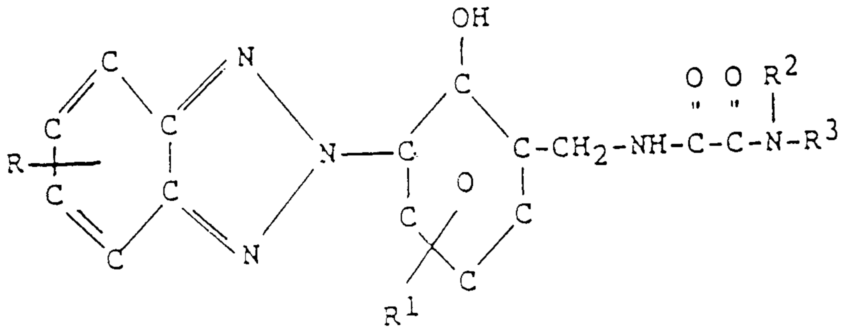

I

## Background of the Invention

### Field of the Invention

This invention relates to compositions of matter classified in the art of chemistry as oxamides which are substituted on one or both nitrogens with a 2-hydroxy-3-(2H-benzotriazol-2-yl)benzyl group, their methods of preparation and their use in stabilizing polymers against the degradative effects of UV light.

### Description of the Prior Art

o-Hydroxyphenyl-2H-benzotriazoles are well known light stabilizers for natural and synthetic polymers. They absorb ultraviolet light over a broad range of the UV spectrum with maximum absorption around 300 and 360nm. However, many of these UV absorbers have limited compatibility in various polymers and show tendencies to exude out of various substrates at high temperatures or to extract out of the substrate and wash away under weathering conditions. Many of the commercial UV absorbers of this class also have tendencies to volatilize or sublime when subjected to the high temperatures required to process engineering resins. Likewise many of the commercial UV absorbers have a tendency to volatilize out of finished products, especially thin films or coatings, when subjected to elevated use temperatures.

Through the years numerous attempts have been made to increase the compatibility of these UV-absorbers with the various substrates and to reduce their volatility by modification of the basic o-hydroxy-2H-benzotriazole structure.

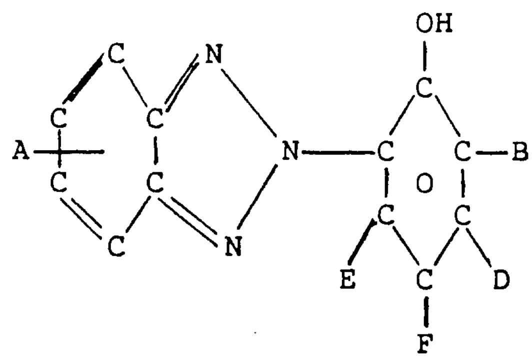

Substitution of larger groups for A, B, D, E or F increased the molecular weight of the benzotriazole and in most cases decreased its volatility and increased its solubility in solvents and polymers. This approach is described in U.S. 4,861,813 which covers 5-alpha-cumyl-2-(2-hydroxy-3,5-di-t-butylphenyl)-2H-benzotriazoles and related compounds. The prior art section of the patent describes additional benzotriazoles containing higher alkyl or aralkyl substituents and their use.

The volatility of o-hydroxy-2-H-benzotriazoles has been decreased by coupling o-hydroxy-2H-benzotriazoles containing functional groups with di or poly functional coreactants. For example, Canadian Patent 1,197,246 teaches the preparation of bis-benzotriazoles by reacting methyl esters of benzotriazolecarboxylic acids with diols or diamines. Tinuvin® 1130, a product of Ciba-Geigy contains the mono and diester products of polyethylene glycol and 3-[3-(benzotriazol-2-yl)-4-hydroxy-5-t-butylphenyl]propionic acid.

U.S. 4,859,726 teaches the preparation of bis-benzotriazolyl compounds of the following structure:

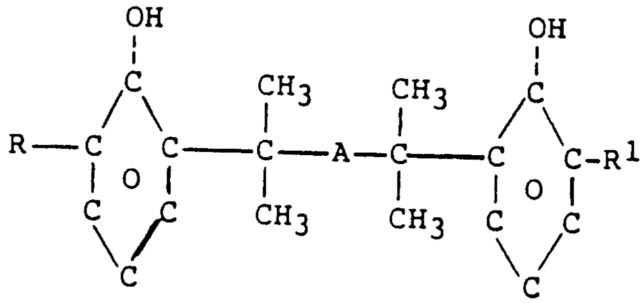

where R and $R^1$ each is a 2- benzotriazolyl radical and A is 1,3- or 1,4- phenylene, by coupling bis phenols of the above structure, where R and $R^1$ are hydrogen, with 2 equivalents of a diazonium salt and then ring closing the resultant o-nitro bis azo compounds to the bis-benzotriazolyl compounds. These compounds have improved resistance to sublimation and/or decomposition when used in synthetic, polymeric materials which are subjected to high temperatures during the processing, casting and/or molding thereof.

Bis[2-hydroxy-3-(benzotriazol-2-yl)phenyl]methane compounds and polymeric materials stabilized therewith are disclosed in U.S. PAT. Nos. 4,681,905, 4,684,679, 4,684,680 and 4,812,498. Bis[2-hydroxy-5-t-octyl-3-(benzotriazol-2-yl)phenyl]methane is currently being offered commercially by Fairmount Chemical Co Inc. as Mixxim® BB/100. It is recommended as a non-volatile UV absorber, particularly useful in engineering resins.

2-Hydroxy-3-(2H-benzotriazol-2-yl)-5-substituted benzyl amides or imides have been prepared by reacting o-hydroxy-2H-benzotriazoles with hydroxymethylamides or hydroxymethylimides respectively (U.S.Pat.Nos. 3,629,192, 3,862,087, 4,612,358 and 4,652,656; Japan Kokai 76 21,587 C.A. 85, 6567s). European Pat. Appl. 0,191,582 covers compounds of the following structure:

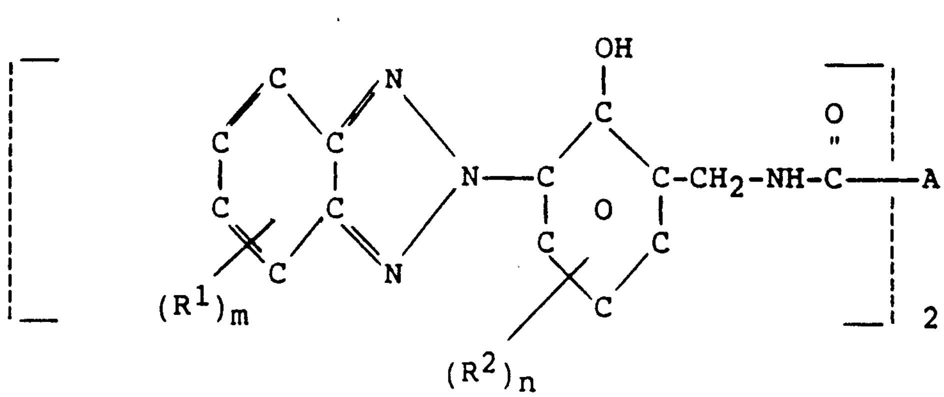

where $R^1$ and $R^2$ are the same or different and each represents a halogen atom, a hydroxy group, an alkyl group with 1-8 carbons or an alkoxy group with 1-18 carbon atoms, A is a phenylene group, a halogen substituted phenylene group or a naphthylene group, m is zero or an integer in the range 1-4, n is zero or an integer in the range 1-3. These compounds were prepared by reacting the o-hydroxy-2H-benzotriazole compounds with the corresponding bis hydroxymethyl aromatic dicarboxamide of the following structure:

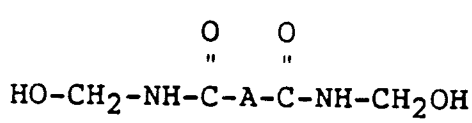

The compounds are claimed to be useful in the stabilization of nylon and polyethylene terephthalate.

To the best of our knowledge there is no reference to any of the compounds of this invention and there is no indication that the compounds of this invention would have enhanced stabilizing properties.

## Definitions

The term "acyl" refers to a radical generated from a carboxylic acid by the removal of the OH group to provide a free valence on the C(=O) group, for example, DC(=O)OH would become the DC(=0) substituent referred to generally as a D acyl group.

The term "oxyl" refers to the oxygen radical as it occurs in the structure ( $>$N—O·).

The terms "polymer" or "polymeric composition(s)" include homopolymers or any type of copolymers.

Where any symbol appears more than once in a formula, its meaning in each instance is independent of one another.

## Summary of the Invention

This invention provides in a principal composition aspect oxamides of Formula I

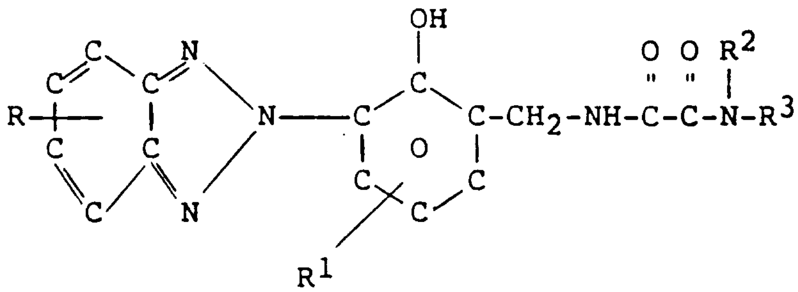

I

wherein:

R may be hydrogen, chlorine, bromine, substituted or unsubstituted aliphatic of 1-8 carbons, substituted or unsubstituted alkoxy of 1-8 carbons, substituted or unsubstituted alkoxycarbonyl of 2-8 carbons, alkylaminocarbonyl of 2-5 carbons, dialkylaminocarbonyl of 3-9 carbons, substituted or unsubstituted N-(alkyl)-N-(aryl)aminocarbonyl of 8-15 carbons, alkoxysulfonyl of 1-4 carbons, -C(=O)-OH, -C(=O)$NH_2$, or -S(=O)$_2$-OH. Preferably, R may be hydrogen, substituted or unsubstituted aliphatic of 1-8 carbons, substituted or unsubstituted alkoxy of 1-8 carbons, methoxycarbonyl, ethoxycarbonyl, carboxy, chloro or S-(=O)$_2$-OH. Most preferably, R may be hydrogen, alkyl of 1-4 carbons, methoxy, ethoxy, chloro, or carboxy.

$R^1$ may be hydrogen, chlorine, bromine, substituted or unsubstituted aliphatic of 1-8 carbons, substituted or unsubstituted aryl of 6-14 carbons, substituted or unsubstituted araliphatic of 7-22 carbons, substituted or unsubstituted alicyclic of 5-12 carbons, substituted or unsubstituted alkoxy of 1-8 carbons, alkylaminocarbonyl of 2-5 carbons, dialkylaminocarbonyl of 3-9 carbons, or substituted or unsubstituted N-(alkyl)-N-(aryl)aminocarbonyl of 8-15 carbons. Preferably, $R^1$ may be hydrogen, substituted or unsubstituted aliphatic of 1-8 carbons, or substituted or unsubstituted araliphatic of 7-9 carbons. Most preferably, $R^1$ may be methyl, ethyl, t-butyl, t-octyl, or 1-methyl-1-phenylethyl.

$R^2$ may be hydrogen, substituted or unsubstituted aliphatic of 1-20 carbons, substituted or unsubstituted araliphatic of 7-22 carbons, or substituted or unsubstituted alicyclic of 5-12 carbons. Preferably, $R^2$ may be hydrogen or lower alkyl of 1-6 carbons. Most preferably, $R^2$ is hydrogen.

$R^3$ may be hydrogen, substituted or unsubstituted aliphatic of 1-20 carbons, substituted or unsubstituted alicyclic of 5-12 carbons, substituted or unsubstituted araliphatic of 7-22 carbons, substituted or unsubstituted aryl of 6-14 carbons, substituted or unsubstituted aromatic or non-aromatic heterocyclic group of 5-12 carbons, where the heteroatom may be oxygen, nitrogen or sulfur, polyoxyalkylene of about 9-44 alkyleneoxide linkages or a radical of Formula II or III

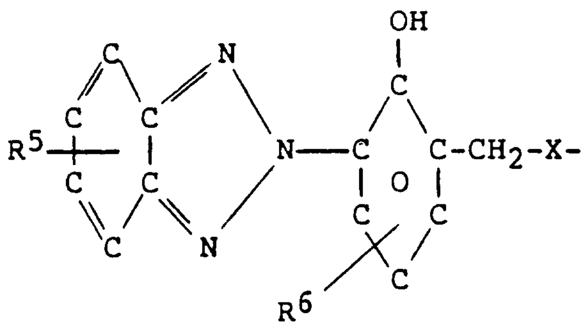

II

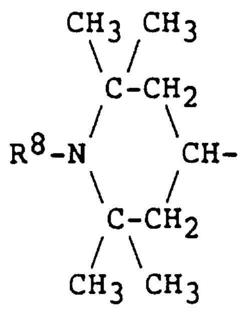

III

wherein X is a direct bond or the diradical:

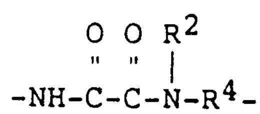

wherein NH is bonded to the methylene of II and $R^4$ is bonded to the oximino nitrogen of I; Preferably, $R^3$ may be hydrogen, substituted or unsubstituted aliphatic of 1-18 carbons, substituted or unsubstituted alicyclic of 6-10 carbons, substituted or unsubstituted araliphatic of 7-15 carbons, substituted or unsubstituted aryl of 6-14 carbons, polyoxyalkylene of about 9-44 alkyleneoxide linkages or a radical of Formula II or III. Most preferably, $R^3$ may be hydrogen, substituted or unsubstituted aliphatic of 3-18 carbons, substituted or unsubstituted alicyclic of 6-8 carbons, substituted or unsubstituted araliphatic of 7-15 carbons, substituted or unsubstituted aryl of 6-14 carbons, polyoxyalkylene of about 9-44 alkyleneoxide linkages or a radical of Formula II.

$R^2$ and $R^3$ may be concatenated to form a substituted or unsubstituted heterocyclic ring including the nitrogen atom to which they are attached of 5-12 carbons or may be concatenated through -N($R^7$)-, -O-, -S- to form a heterocyclic ring including the nitrogen to which they are attached of 6-12 atoms wherein heteroatoms are separated by at least two carbon atoms. Preferably, when $R^2$ and $R^3$ are concatenated they may form a substituted or unsubstituted piperidine, piperazine, or morpholine ring. Most preferably, when $R^2$ and $R^3$ are concatenated they form a piperidine ring.

$R^4$ may be a substituted or unsubstituted aliphatic diradical of 1-20 carbons, substituted or unsubstituted aryl diradical of 6-12 carbons, substituted or unsubstituted alicyclic diradical of 5-12 carbons, or substituted or unsubstituted araliphatic diradical of 7-22 carbons, the aliphatic chains of which may optionally contain heteroatoms -O-, -S- and -N($R^7$)- with the proviso that multiple heteroatoms must be

separated from each other and the diradical ends by at least two carbon atoms or $R^4$ may also be a polyoxyalkylene diradical of about 3-34 alkyleneoxide linkages. Preferably, $R^4$ may be a substituted or unsubstituted aliphatic diradical of 2-12 carbons or a 1,3- or 1,4-phenylene diradical. Most preferably, $R^4$ is an alkylene diradical of 2-10 carbons.

$R^5$ may be hydrogen, chlorine, bromine, substituted or unsubstituted aliphatic of 1-8 carbons, substituted or unsubstituted alkoxy of 1-8 carbons, substituted or unsubstituted alkoxycarbonyl of 2-8 carbons, -C(=O)-OH, -C(=O)$NH_2$, or -S(=O)$_2$-OH.Preferably, $R^5$ may be hydrogen, substituted or unsubstituted aliphatic of 1-8 carbons, substituted or unsubstituted alkoxy of 1-8 carbons, methoxycarbonyl, ethoxycarbonyl, carboxy, chloro or S(=O)$_2$-OH. Most preferably, $R^5$ may be hydrogen, alkyl of 1-4 carbons, methoxy, ethoxy, chloro, or carboxy.

$R^6$ may be hydrogen, substituted or unsubstituted aliphatic of 1-8 carbons, substituted or unsubstituted aryl of 6-14 carbons, substituted or unsubstituted araliphatic of 7-22 carbons or substituted or unsubstituted alkoxy of 1-8 carbons. Preferably, $R^6$ may be hydrogen, substituted or unsubstituted aliphatic of 1-8 carbons, or substituted or unsubstituted araliphatic of 7-9 carbons. Most preferably, $R^6$ may be methyl, ethyl, tert.-butyl, tert.-octyl, or 1-methyl-1-phenylethyl.

$R^7$ may be hydrogen, substituted or unsubstituted aliphatic of 1-20 carbons, substituted or unsubstituted alicyclic of 5-12 carbons, substituted or unsubstituted araliphatic of 7-22 carbons, substituted or unsubstituted aliphatic acyl of 2-20 carbons, substituted or unsubstituted alicyclic acyl of 7-16 carbons, substituted or unsubstituted araliphatic acyl of 8-23 carbons or substituted or unsubstituted aryl acyl of 7-11 carbons. Preferably $R^7$ may be hydrogen, substituted or unsubstituted araliphatic of 7-10 carbons, substituted or unsubstituted aliphatic acyl of 2-6 carbons or substituted or unsubstituted benzoyl. Most preferably $R^7$ may be hydrogen, methyl, acetyl or benzoyl.

$R^8$ may be hydrogen, oxyl, alkyl of 1-8 carbons, alkenyl or alkynyl of 3-12 carbons, aliphatic acyl of 1-12 carbons, aromatic acyl of 7-15 carbons, aralkyl of 7-9 carbons or hydroxyalkyl of 2-6 carbons. Preferably, $R^8$ may be hydrogen, alkyl of 1-4 carbons, alkenyl of 3 or 4 carbons, benzyl, acetyl or benzoyl. Most preferably, $R^8$ may be hydrogen, methyl, acetyl or benzoyl.

Optional substitutents for R, $R^1$, $R^2$, $R^3$, $R^4$, $R^5$, $R^6$ and $R^7$ may be independently one or more of the following: amino, alkylamino of 1-4 carbons, dialkylamino of 2-8 carbons, chloro, bromo, alkyl of 1-8 carbons, alkoxy of 1-8 carbons, phenoxy, cyano, hydroxy, epoxy, carboxy, alkoxycarbonyl of 2-6 carbons, acryloyl, methacryloyl, acyloxy of 1-6 carbons, acryloyloxy, methacryloyloxy, hydroxymethyl, hydroxyethyl, alkylthio of 1-4 carbons, or trialkoxysilyl of 3-12 carbons.

The invention provides in a second principal composition aspect a light stabilized polymeric composition comprising at least one oxamide of Formula I and a natural or synthetic polymer.

The invention provides in a first process aspect a process for the manufacture of oxamides of Formula I wherein $R^2$ is hydrogen and $R^3$ is not an aryl group which comprises:

a) treating an aminomethyl benzotriazole of Formula IV:

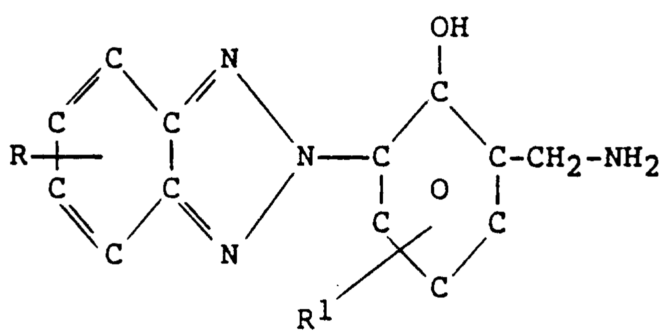

IV

wherein R and $R^1$ are as previously defined, with an alkyl oxalyl chloride of the formula:

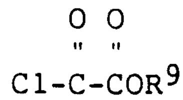

wherein $R^9$ is lower alkyl of 1 to 4 carbon atoms or allyl in the presence of an acid acceptor to provide oxamate esters of Formula V:

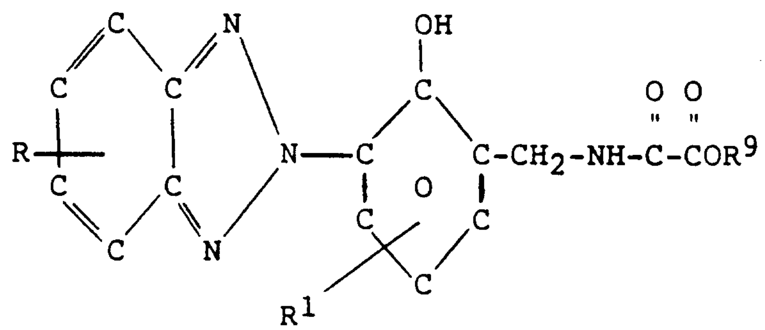

V

wherein $R^9$ is as previously defined; or

a') in the alternative, oxamate esters of Formula Va are prepared:

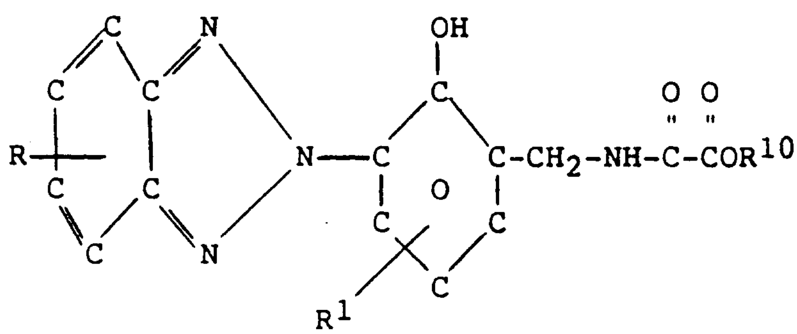

Va

wherein R and $R^1$ are are as previously defined and $R^{10}$ is lower alkyl of 1-4 carbon atoms or phenyl by treating a benzotriazole of Formula IV

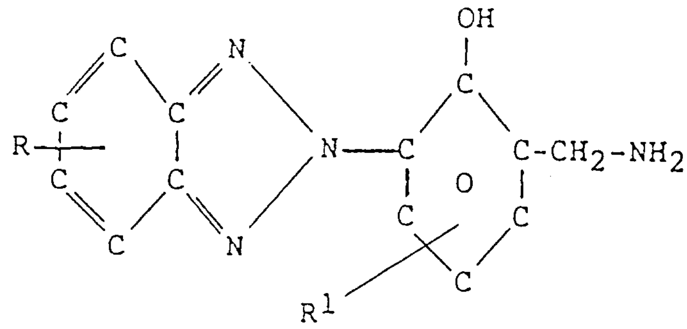

IV

wherein R and $R^1$ are as previously defined with a lower dialkyl oxalate or diphenyl oxalate of the formula:

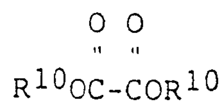

wherein $R^{10}$ is as defined herein above; and

b) treating the oxamate esters V or Va with an amine of the structure:

$R^3NH_2$

wherein $R^3$ is aliphatic, cycloaliphatic, heterocyclic or polyoxyalkylene, all as further defined hereinabove to form a compound of Formula I wherein $R^2$ is hydrogen and $R^3$ is not an aryl group (preferably R = H, $R^1$ = methyl and para to the hydroxy group and $R^3$ = substituted or unsubstituted aliphatic of 3 -18 carbons, cyclohexyl or polyoxyalkylene as defined above);

b') the oxamate esters of formulas V or Va are treated with primary, cycloaliphatic, araliphatic or polyoxyalkylene diamines having the structure

$H_2N\text{-}R^4\text{-}NH_2$

wherein $R^4$ is as defined hereinabove to provide a structure of formula I wherein $R^2$ is hydrogen, $R^4$ is not an aryl diradical and $R^3$ is Formula II wherein X is

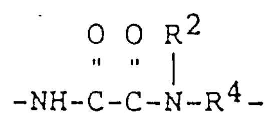

or

$R^3$ is $-R^4\text{-}NH_2$. It is to be understood that all processes for the preparation of oxamides with two 2-(2-hydroxyphenyl)-2H-benzotriazolemoieties, i.e. oxamides ox Formula I with $R^3$ being Formula II, are limited to the preparation of those oxamides in which one benzotriazole moiety bears R and $R^1$ and the other benzotriazole moiety bears $R^5$ and $R^6$;

b'') in a first alternative, the oxamate esters of Formulas V or Va are treated with the same or a different structural benzotriazole of Formula IV that was employed to form the compound of Formula V or Va to form compounds of Formula I wherein $R^3$ is a radical of Formula II wherein X is a direct bond and wherein $R^2$ is hydrogen; or

b''') in a second alternative, the oxamate esters of Formula V or Va are treated with 4-amino substituted 2,2,6,6-tetramethylpiperidine to form compounds of Formula I wherein $R^3$ is a radical of Formula III and wherein $R^2$ is hydrogen.

The invention provides in a second process aspect, a process for the preparation of compounds of Formula I which comprises:

a) treating a lower alkyl oxalyl chloride having the structure

$$R^{9}O\overset{\overset{O}{\|}}{C}-\overset{\overset{O}{\|}}{C}-Cl$$

wherein $R^9$ is as previously defined, with an amine of the formula:

$R^2R^3NH$

wherein $R^2$ and $R^3$ are as defined for Formula I to produce a compound of Formula VI

$$R^{9}O\overset{\overset{O}{\|}}{C}-\overset{\overset{O}{\|}}{C}-NR^{2}R^{3}$$

VI

wherein $R^9$, $R^2$ and $R^3$ are as defined hereinabove; or

a') in the alternative, treating a di-lower alkyl oxalate or diphenyl oxalate of the formula:

$$R^{10}O\overset{\overset{O}{\|}}{C}-\overset{\overset{O}{\|}}{C}-OR^{10}$$

wherein $R^{10}$ is as previously defined with an amine of the formula:

$R^2R^3NH$

wherein $R^2$ and $R^3$ are as defined for Formula I to provide a compound of the Formula VIa:

$$R^{10}O\overset{\overset{O}{\|}}{C}-\overset{\overset{O}{\|}}{C}-NR^{2}R^{3}$$

VIa

wherein $R^{10}$, $R^2$ and $R^3$ are as defined hereinabove; and

b) treating VI or VIa with an aminobenzotriazole of Formula IV to produce a compound of Formula I.

The invention provides in a third process aspect a process for producing compounds of Formula I which comprises:

a) treating a lower alkyl oxalyl chloride having the formula

$$R^{9}O\overset{\overset{O}{\|}}{C}-\overset{\overset{O}{\|}}{C}-Cl$$

wherein $R^9$ is as previously defined with a diamine having the formula

$HN(R^2)-R^4-N(R^2)H$

wherein $R^2$ and $R^4$ are as defined for Formula I to provide a compound of the Formula VII

```
   O O                        O O
   " "                        " "
R⁹OC-C-N(R²)-R⁴-N(R²)-C-COR⁹
```

VII

wherein $R^2$, $R^4$ and $R^9$ are as previously defined; or

(a') in the alternative, treating a di-lower alkyl oxalate or diphenyl oxalate having the formula

```
     O O
     " "
R¹⁰OC-COR¹⁰
```

wherein $R^{10}$ is as previously defined with a diamine having the structure $HN(R^2)-R^4-N(R^2)H$ wherein $R^2$ and $R^4$ are as previously defined to provide a compound having the Formula VIIa

```
    O O                        O O
    " "                        " "
R¹⁰OC-C-N(R²)-R⁴-N(R²)-C-COR¹⁰
```

VIIa

wherein $R^2$, $R^4$ and $R^{10}$ are as previously defined; and

b) treating a compound of Formula VII or VIIa with an aminomethyl benzotriazole to provide a compound of Formula I wherein $R^3$ is a radical of Formula II

wherein X is the diradical

```
     O O   R²
     " "   |
-NH-C-C —N-R⁴-
```

wherein $R^2$ and $R^4$ are as previously defined.

The invention provides in a fourth composition aspect a process for the preparation of compounds of Formula I wherein $R^2$ is hydrogen, $R^3$ is a radical of Formula II and X is a direct bond which comprises treating a 2-(2-hydroxyphenyl)-2H-benzotriazole of the Formula VIII

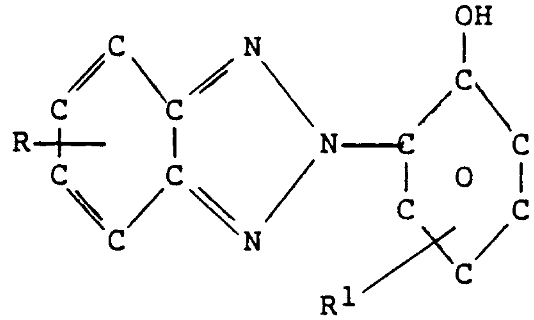

VIII

wherein R and $R^1$ are as defined for Formula I with N,N'-bis(hydroxymethyl)oxamide to provide a compound of Formula I having the formula Ia:

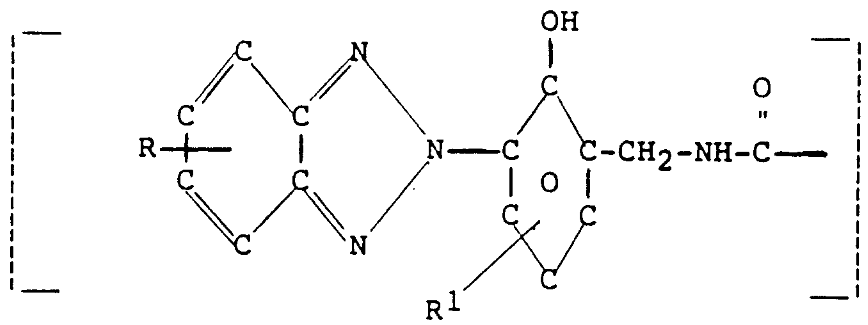

Ia

The invention also provides in a second composition aspect a polymer composition stabilized against light induced degradation which comprises a polymer selected from the group consisting of natural polymers, synthetic polymers and mixtures thereof and a stabilizing effective amount of a compound of Formula I.

Detailed Description of the Preferred Embodiments

General Formula

The novel compounds of this invention are oxamides containing [2-hydroxy-3-(benzotriazol-2-yl)benzyl] groups on one or both of the oxamide nitrogens. They have the following general formula:

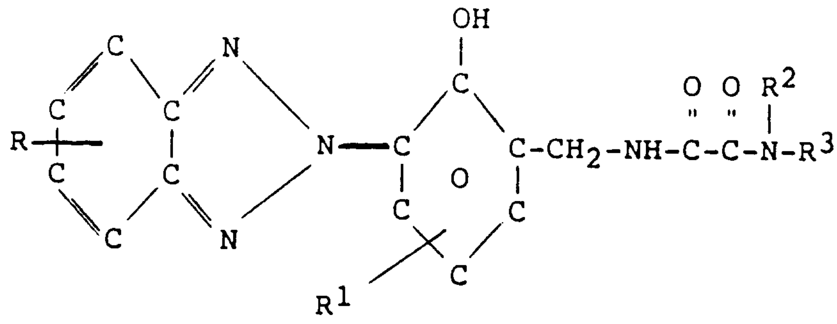

I

where R, $R^1$, $R^2$, $R^3$ and $R^4$ and all their substitutents are as previously defined.

Generic Group Examples

As substituted or unsubstituted aliphatic of 1-8 carbons, R, $R^1$, $R^5$, and $R^6$ may be, independently, for example, methyl, 2-hydroxyethyl, ethyl, 2-acetoxyethyl, isopropyl, 2-chloroethyl, allyl, methallyl, pentyl, 2-(methacryloyloxy)ethyl, 3-pentyl, tert.-butyl, hexyl, octyl, or tert.-octyl.

As substituted or unsubstituted aliphatic of 1-20 carbons, $R^2$ and $R^7$ may be, independently, for example, methyl, ethyl, n-propyl, isopropyl, butyl, allyl, n-pentyl, 2-bromoethyl, hexyl, heptyl, octyl, nonyl, decyl, propargyl, octadecyl, dodecyl, isododecyl, 2-acetoxyethyl, tetradecyl, 2-methallyl, 2-hexenyl, 10-undecenyl, 2-dodecenyl, n-butyl, 2-hydroxyethyl, 2-butenyl, 2-hydroxyhexadecyl, 2-hydroxypropyl, 2-hexenyl, 10-undecenyl, 2-hydroxydodecyl, 2-hydroxy-5-hexenyl, 2-hydroxyhexyl, 2-hydroxydecyl, 2-hydroxyoctadecyl, 2-hydroxy-3-(methacryloyloxy)propyl, 2-hydroxy-3-(acryloyloxy)propyl, 2-hydroxy-3-phenoxypropyl, 2-hydroxy-3-(4-methoxyphenoxy)propyl, 2-hydroxy-3-isopropoxypropyl, 2-hydroxy-3-methoxypropyl, 2-hydroxy-3-(2-ethylhexoxy)propyl, 3-(trimethoxysilyl)propyl, 2-hydroxy-3-(cyclohexoxy)propyl, 2-hydroxy-3-(benzoxy)propyl, 2-hydroxy-3-(benzoyloxy)propyl, 2-hydroxy-3-dodecoxypropyl, 2-hydroxybutyl, 1-methyl-2-hydroxypropyl, cyanomethyl, 2,3-epoxypropyl, or 2-(dimethylamino)ethyl, 2-aminoethyl, 4-aminobutyl or 6-aminohexyl.

As substituted or unsubstituted alkoxy of 1-8 carbons, R, $R^1$, $R^5$ and $R^6$ may be, independently, for example, methoxy, ethoxy, 2-ethylhexoxy, isopropoxy, 2-hydroxypropoxy, 2-(acryloyloxy)ethoxy, or sec-butoxy.

As substituted or unsubstituted alkoxycarbonyl of 2-8 carbons, R and $R^5$ may be, for example, methoxycarbonyl, ethoxycarbonyl, 2-hydroxyethoxycarbonyl, allyloxycarbonyl, or butoxycarbonyl.

As alkylaminocarbonyl of 2-5 carbons, dialkylaminocarbonyl of 3-9 carbons and substituted or unsubstituted N-(aryl)-N-(alkyl)aminocarbonyl of 8-15 carbons, R and $R^1$ may be, independently, for example, methylaminocarbonyl, ethylaminocarbonyl, butylaminocarbonyl, N-(4-methylphenyl)-N-methylaminocarbonyl, N-phenyl-N-methylaminocarbonyl, N-(2-ethoxyphenyl)-N-ethylaminocarbonyl, N-(3-isopropenylphenyl)-N-butylaminocarbonyl, dimethylaminocarbonyl, or dibutylaminocarbonyl.

As alkoxysulfonyl of 1-4 carbons, R may be, for example, methoxysulfonyl or butoxysulfonyl.

As substituted or unsubstituted alicyclic of 5-12 carbons, $R^2$, $R^3$, and $R^7$ may be, independently, for example, cyclohexyl, trimethylcyclohexyl, cyclooctyl, cyclododecyl, 4-tert.-butylcyclohexyl, 2-hydroxycyclododecyl, 3-cyclohexenyl, 2-hydroxycyclohexyl, 2-hydroxycyclopentyl, cyclododecyl, 4-octylcyclohexyl, or 2-methyl-4-octylcyclohexyl.

As substituted or unsubstituted aryl of 6-14 carbons, $R^1$, $R^3$ and $R^6$ may be, independently, for example, phenyl, tolyl, 4-chlorophenyl, isopropylphenyl, isopropenylphenyl, anisyl, 3,5-di-tert.-butyl-4-hydroxyphenyl, 3,5-di-tert.-amyl-4-hydroxyphenyl, 4-vinylphenyl, 3-tert.-butyl-5-methyl-4-hydroxyphenyl, naphthyl, 3-methyl-5-tert.-butyl-4-hydroxyphenyl, 3,4,5-trimethoxyphenyl, 4-aminophenyl, 4-(dimethylamino)-phenyl, 2-methoxyphenyl, 2-ethoxyphenyl or 2-isopropoxyphenyl.

As substituted or unsubstituted araliphatic of 7-22 carbons, $R^1$, $R^2$, $R^3$, $R^6$ and $R^7$ may be, independently, for example, benzyl, 3-methylbenzyl, 4-tert.-butylbenzyl, cinnamyl, 3,5-di-tert.-butyl-4-hydroxybenzyl, 2-hydroxy-2-phenylethyl, 2-phenylethyl, cumyl, trimethylbenzyl, 4-octoxybenzyl, naphthylmethyl, (4-dodecylphenyl)methyl, 2-(3,5-di-tert.-butyl-4-hydroxyphenyl)ethyl, 2-(3,5-di-tert.-amyl-4-hydroxyphenyl)ethyl, or 2-(3-tert.-butyl-5-methyl-4-hydroxyphenyl)ethyl.

As a substituted or unsubstituted aromatic or non-aromatic heterocyclic group of 5-12 carbons, $R^3$ may be, for example, 4-oxacyclohexyl, 4-thiacyclohexyl, 4-azacyclohexyl, 5-oxacyclooctyl or pyridin-4-yl.

As a polyoxyalkylene group of about 9-44 alkyleneoxide linkages, $R^3$ may be, for example, polyoxyethylene of about 12-13 ethyleneoxide linkages (derived from Texaco Chemical Co. Jeffamine® M-600) or polyoxyalkylene containing about 3-4 ethyleneoxide linkages and about 18 propyleneoxide linkages (derived from Texaco Chemical Co. Jeffamine® M-1000).

As a radical of Formula II, $R^3$ may be, for example, 2-hydroxy-3-(2H-benzotriazol-2-yl)-5-methylbenzyl-, 2-hydroxy-3-(2H-benzotriazol-2-yl)-5-tert.-butylbenzyl-, 2-hydroxy-3-(2H-5-chlorobenzotriazol-2-yl)-5-tert.-butylbenzyl or 2-hydroxy-3-(2H-5-chlorobenzotriazol-2-yl)-5-methylbenzyl.

As a radical of formula III, $R^3$ may be, for example, 2,2,6,6-tetramethylpiperidin-4-yl, 1-allyl-2,2,6,6-tetramethylpiperidin-4-yl, 1,2,2,6,6-pentamethylpiperidin-4-yl, 1-benzoyl-2,2,6,6-tetramethylpiperidin-4-yl or 1-(2-hydroxyethyl)-2,2,6,6-tetramethylpiperidin-4-yl.

As a substituted or unsubstituted heterocyclic ring, $R^2$ and $R^3$ when concatenated, may form for example, piperazinyl, N-methylpiperazinyl, N-2-hydroxyethylpiperazinyl, piperidinyl or morpholinyl rings.

As a substituted or unsubstituted aliphatic diradical of 1-20 carbons, substituted or unsubstituted aryl diradical of 6-12 carbons, substituted or unsubstituted alicyclic diradical of 5-12 carbons and substituted or unsubstituted araliphatic diradical of 7-22 carbons, $R^4$ may be, for example, methylene, 1,2-ethanediyl, 1,2-propanediyl, 1,3-propanediyl, 1,4-butanediyl, 1,18-octadecanediyl, 2,2-dimethyl-1,3-propanediyl, 2-methylpentane-2,4-diyl, 1,10-decanediyl, 1,12-dodecanediyl, 3-oxapentane-1,5-diyl, 4-oxaheptane-1,7-diyl, 3,6-dioxaoctane-1,8-diyl, 4,9-dioxadodecane-1,12-diyl, 4-methyl-4-azaheptane-1,4-diyl, 3,6-diaza-3,6-dimethyl-1,8-octanediyl, 3-methyl-3-azapentane-1,5-diyl, 1,2-cyclohexanediyl, 1,4-cyclohexanediyl, 1,2-ethenediyl, 1,2-propenediyl, 1-chloro-1,2-ethenediyl, 1-phenyl-1,2-ethenediyl, 1,3-hexanediyl, 1,2-cyclohexanediyl, 4-methyl-4-cyclohexene-1,2-diyl, 4-cyclohexene-1,2-diyl, 4-methylcyclohexane-1,2-diyl, 4-carboxy-1,2-phenylene, 4-methoxycarbonyl-1,2-phenylene, propane-2,2-bis(4-cyclohexyl), 3-oxapentane-1,5-diyl, methylenebis(4-cyclohexyl), 1,2-, 1,3-, or 1,4-phenylene, 1,2-, 1,3-, or 1,4-phenylenebis(methyl), biphenyl-4,4'-diyl, biphenyl-3,3'-diyl, biphenyl-3,4'-diyl, or methylenebis(phenylene).

As a polyoxyalkylene diradical of about 3-34 alkyleneoxide linkages, $R^4$ may be, for example, polyoxypropylene of about 3-4 propyleneoxide linkages (derived from Texaco Chemical Co. Jeffamine® D-230) or polyoxypropylene of about 34 propyleneoxide linkages (derived from Texaco Chemical Co. Jeffamine® D-2000).

As a substituted or unsubstituted aliphatic acyl of 2-20 carbons, $R^7$ may be, for example, acetyl, propionyl, acryloyl, methacryloyl, stearoyl or chloroacetyl.

As a substituted or unsubstituted alicyclic acyl of 7-16 carbons, $R^7$ may be, for example, cyclohexanecarbonyl, cycloheptanecarbonyl, 4-tert.-butylcyclohexanecarbonyl or 3,5,5-trimethylcyclohexanecarbonyl.

As a substituted or unsubstituted araliphatic acyl of 8-23 carbons, $R^7$ may be, for example, cinnamoyl, dihydrocinnamoyl, 3-(3,5-di-tert.-butyl-4-hydroxyphenyl)propionyl, 2-(3,5-di-tert.-butyl-4-hydroxyphenyl)-acetyl, 3-(3-tert.-butyl-4-hydroxy-5-methylphenyl)propionyl or phenylacetyl.

As aminoalkyl of 1-4 carbons, the optional substituents may be, for example, aminomethyl, 2-aminoethyl, 3-aminopropyl, 2-aminopropyl, 2-amino-2-propyl, 4-aminobutyl or 2-aminobutyl.

As dialkylamino of 2-8 carbons, the optional substituents may be, for example, dimethylamino, diethylamino, dipropylamino, dibutylamino, N-methyl-N-propylamino or N-methyl-N-butylamino.

As alkoxycarbonyl of 2-6 carbons, the optional substituents may be, for example, methoxycarbonyl, ethoxycarbonyl, isopropoxycarbonyl or butoxycarbonyl.

As acyloxy of 1-6 carbons, the optional substituents may be, for example, acetoxy, propionoxy, butyroyloxy, valeroyloxy or hexanoyloxy.

As alkylthio of 1-4 carbons, the optional substituents may be, for example, methylthio, ethylthio, propylthio, isopropylthio, butylthio or tert.-butylthio.

As trialkoxysilyl of 3-12 carbons, the optional substituents may be, for example, trimethoxysilyl, triethoxysilyl, triisopropoxysilyl or tributoxysilyl.

## List of Example Compounds

Examples of stabilizer compounds of the present invention include the following non-limiting list of compounds:

1. N-[2-hydroxy-3-(2H-5-chlorobenzotriazol-2-yl)-5-tert.-pentylbenzyl]-N'-hexadecyloxamide
2. N-[2-hydroxy-3-(2H-5-bromobenzotriazol-2-yl)-5-tert.-cumylbenzyl]-N'-tetradecyloxamide
3. N-[2-hydroxy-3-(2H-5-octylbenzotriazol-2-yl)-5-methylbenzyl]oxamide
4. N-[2-hydroxy-3-(2H-5-methoxybenzotriazol-2-yl)-5-ethylbenzyl]-N'-cyclohexyl-N'-cyclopentyloxamide

5. N-[2-hydroxy-3-(2H-5-butoxybenzotriazol-2-yl)-5-propylbenzyl]-N',N'-dicyclooctyloxamide
6. N-[2-hydroxy-3-(2H-5-hexoxybenzotriazol-2-yl)-5-isopropylbenzyl]-N'-2-hydroxycyclohexyl-N'-cyclododecyloxamide
7. N-[2-hydroxy-3-(2H-5-methoxycarbonylbenzotriazol-2-yl)-5-phenylbenzyl]-N'-methyl-N'-4-tert.-butylcyclohexyloxamide
8. N-{2-hydroxy-3-[2H-5-(ethoxycarbonyl)benzotriazol-2-yl]-5-p-(methylphenyl)benzyl}-N'-ethyl-N'-tert.-cumyloxamide
9. N-{2-hydroxy-3-[2H-5-(methylaminocarbonyl)benzotriazol-2-yl]-5-methoxybenzyl}-N'-propyl-N'-benzyloxamide
10. N-{2-hydroxy-3-[2H-5-(ethylaminocarbonyl)benzotriazol-2-yl]-5-chlorobenzyl}-N'-butyl-N'-phenyloxamide
11. N-{2-hydroxy-3-[2H-5-(dimethylaminocarbonyl)benzotriazol-2-yl]-5-(dimethylaminocarbonyl)benzyl}-N',N'-di-(2-hydroxyethyl)oxamide
12. N-{2-hydroxy-3-[2H-5-(diethylaminocarbonyl)benzotriazol-2-yl]-benzyl}-N'-methyl-N'-2-hydroxyethyloxamide
13. N-{2-hydroxy-3-[2H-5-(N-methyl-N-phenylaminocarbonyl)benzotriazol-2-yl]-5-(N-methyl-N-phenylaminocarbonyl)benzyl}-N'-benzyl-N'-(o-isopropoxyphenyl)oxamide
14. N-{2-hydroxy-3-[2H-5-(N-butyl-N-phenylaminocarbonyl)benzotriazol-2-yl]-5-(N-butyl-N-phenylaminocarbonyl)benzyl}-N'-2-phenylethyl-N'-(2-aminoethyl)oxamide
15. N-{2-hydroxy-3-[2H-5-(methoxysulfonyl)benzotriazol-2-yl]-5-ethoxybenzyl}-N'-3-(3,5-di-tert.-butyl-4-hydroxyphenyl)propyl-N'-(4-aminobutyl)oxamide
16. N-{2-hydroxy-3-[2H-5-(butoxysulfonyl)benzotriazol-2-yl]-5-(acetoxyethyl)benzyl}-N'-(6-aminohexyl)-oxamide
17. N-[2-hydroxy-3-(2H-5-carboxybenzotriazol-2-yl)-5-bromobenzyl]-N'-methyl-N'-(p-aminophenyl)-oxamide
18. N-[2-hydroxy-3-(2H-5-carboxybenzotriazol-2-yl)-5-octylbenzyl]-N'-(p-ethoxyphenyl)oxamide
19. N-[2-hydroxy-3-(2H-5-amidobenzotriazol-2-yl)-5-(2-methacryloyloxyethyl)benzyl]-N'-[3-(triethoxysilyl)-propyl]oxamide
20. N-[2-hydroxy-3-(2H-5-amidobenzotriazol-2-yl)-5-(sec.butoxy)benzyl]-N'-4-oxacyclohexyloxamide
21. N-[2-hydroxy-3-(2H-5-sulfobenzotriazol-2-yl)-5-(3,5-di-tert.-butyl-4-hydroxyphenyl)benzyl]-N'-pyridin-4-yloxamide
22. N-[2-hydroxy-3-(2H-5-ethylbenzotriazol-2-yl)-5-naphthylbenzyl]-N'-cyclohexyloxamide
23. N-[2-hydroxy-3-(2H-5-tert.-butylbenzotriazol-2-yl)-5-(p-chlorophenyl)benzyl]-N'-piperidin-4-yloxamide
24. N-[2-hydroxy-3-(2H-5-allylbenzotriazol-2-yl)-5-allylbenzyl]-N'-(2,2,6,6-tetramethylpiperidin-4-yl)-oxamide
25. N-[2-hydroxy-3-(2H-5-isopropylbenzotriazol-2-yl)-5-(p-methoxyphenyl)benzyl]-N'-(1-acetyl-2,2,6,6-tetramethylpiperidin-4-yl)oxamide
26. N-{2-hydroxy-3-[2H-5-(2-hydroxyethyl)benzotriazol-2-yl]-5-benzylbenzyl}-N'-(1-allyl-2,2,6,6-tetramethylpiperidin-4-yl)oxamide
27. N-[2-hydroxy-3-(2H-5-methallylbenzotriazol-2-yl)-5-(alpha-methylbenzyl)benzyl]-N'-methallyloxamide
28. N,N'-Bis[2-hydroxy-3-(2H-5-chlorobenzotriazol-2-yl)-5-methylbenzyl]oxamide
29. N,N'-Bis[2-hydroxy-3-(2H-benzotriazol-2-yl)-5-tert.-butylbenzyl]oxamide
30. N,N'-Bis[2-hydroxy-3-(2H-5-chlorobenzotriazol-2-yl)-5-tert.-butylbenzyl]oxamide
31. N,N'-Bis[2-hydroxy-3-(2H-benzotriazol-2-yl)-4-octylbenzyl]oxamide
32. N,N'-Bis[2-hydroxy-3-(2H-benzotriazol-2-yl)-5-ethylbenzyl]oxamide
33. N,N'-Bis[2-hydroxy-3-(2H-benzotriazol-2-yl)-5-isopropylbenzyl]oxamide
34. N,N'-Bis[2-hydroxy-3-(2H-5-bromobenzotriazol-2-yl)-5-methylbenzyl]oxamide
35. N,N'-Bis[2-hydroxy-3-(2H-benzotriazol-2-yl)-5-tert.-amylbenzyl]oxamide
36. N,N'-Bis[2-hydroxy-3-(2H-5-chlorobenzotriazol-2-yl)-5-tert-amylbenzyl]oxamide
37. N-[2-hydroxy-3-(2H-benzotriazol-2-yl)-5-tert.-octylbenzyl]oxamide
38. N-[2-hydroxy-3-(2H-5-chlorobenzotriazol-2-yl)-5-methylbenzyl]oxamide
39. N-[2-hydroxy-3-(2H-5-chlorobenzotriazol-2-yl)-5-tert.-butylbenzyl]oxamide
40. N-[2-hydroxy-3-(2H-benzotriazol-2-yl)-5-tert.-cumylbenzyl]oxamide
41. N,N'-ethylenebis{N'-[2-hydroxy-3-(2H-benzotriazol-2-yl)-5-methylbenzyl]oxamide}
42. N,N'-ethylenebis{N'-[2-hydroxy-3-(2H-5-chlorobenzotriazol-2-yl)-5-methylbenzyl]oxamide}
43. N,N'-1,4-butylenebis{N'-[2-hydroxy-3-(2H-5-bromobenzotriazol-2-yl)-5-ethylbenzyl]oxamide}
44. N,N'-1,6-hexylenebis{N'-[2-hydroxy-3-(2H-5-methylbenzotriazol-2-yl)-5-tert.-octylbenzyl]oxamide}
45. N,N'-1,4-phenylenebis{N'-[2-hydroxy-3-(2H-5-octylbenzotriazol-2-yl)-5-tert.-octylbenzyl]oxamide}

46. N,N'-1,3-phenylenebis{N'-[2-hydroxy-3-(2H-benzotriazol-2-yl)-5-tert.-octylbenzyl]oxamide}
47. N,N'-1,12-dodecylenebis{N'-[2-hydroxy-3-(2H-benzotriazol-2-yl)-5-methylbenzyl]oxamide}
48. N,N'-1,4-cyclohexylenebis{N'-[2-hydroxy-3-(2H-benzotriazol-2-yl)-5-ethylbenzyl]oxamide}
49. N-[2-hydroxy-3-(2H-benzotriazol-2-yl)-5-methylbenzyl]-N'-[2-hydroxy-3-(2H-benzotriazol-2-yl)-5-tert.-octylbenzyl]oxamide
50. N-[2-hydroxy-3-(2H-5-chlorobenzotriazol-2-yl)-5-methylbenzyl]-N'-[2-hydroxy-3-(2H-benzotriazol-2-yl)-5-methylbenzyl]oxamide
51. N-[2-hydroxy-3-(2H-5-chlorobenzotriazol-2-yl)-5-methylbenzyl]-N'-[2-hydroxy-3-(2H-benzotriazol-2-yl)-5-tert.-octylbenzyl]oxamide
52. N-[2-hydroxy-3-(2H-5-chlorobenzotriazol-2-yl)-5-tert.-butyl-benzyl]-N'-[2-hydroxy-3-(2H-benzotriazol-2-yl)-5-tert.-octylbenzyl]oxamide
53. N-[2-hydroxy-3-(2H-benzotriazol-2-yl)-5-tert.-butylbenzyl]-N'-[2-hydroxy-3-(2H-benzotriazol-2-yl)-5-methylbenzyl]oxamide
54. N-[2-hydroxy-3-(2H-benzotriazol-2-yl)-5-tert.-butylbenzyl]-N'-[2-hydroxy-3-(2H-benzotriazol-2-yl)-5-tert.-octylbenzyl]oxamide
55. N-[2-hydroxy-3-(2H-5-chlorobenzotriazol-2-yl)-5-tert.-butylbenzyl]-N'-[2-hydroxy-3-(2H-benzotriazol-2-yl)-5-methylbenzyl]oxamide
56. N-[2-hydroxy-3-(2H-5-methoxybenzotriazol-2-yl)-5-methylbenzyl]-N'-[2-hydroxy-3-(2H-benzotriazol-2-yl)-5-tert.-octylbenzyl]oxamide
57. N-[2-hydroxy-3-(2H-5-methoxybenzotriazol-2-yl)-5-methylbenzyl]-N'-[2-hydroxy-3-(2H-5-methoxybenzotriazol-2-yl)-5-tert.-octyl-benzyl]oxamide

## UTILITY

The novel N-[2-hydroxy-3-(2H-benzotriazol-2-yl)benzyl]oxamides of this invention possess the inherent applied use characteristic of being very effective additives for the stabilization of polymeric compositions which are normally subject to thermal and actinic light degradation. They are especially effective light stabilizers for polycarbonate, nylon, polyethylene terephthalate, polybutylene terephthalate and poly-(phenylene oxides). The 2-hydroxy-3-(2H-benzotriazol-2-yl)benzyl groups are efficient ultraviolet light absorbing groups. It is believed that they convert the absorbed actinic energy into non-destructive heat through a series of tautomeric shifts. The oxamide group in the structure contributes some additional light stabilizing properties and also provides heat stabilizing and metal complexing properties to the novel compounds. When the oxamide group is substituted on the other nitrogen with an aryl group, the aryl substituted oxamide group enhances the UV absorbing properties in the 290 to 310 nm region of the spectra.

The compounds of this invention have low volatility in the temperature range (250-300 °C) required for processing engineering resins such as polycarbonate (PC), nylon, polyethylene terephthalate (PET), polybutylene terephthalate (PBT), and polyphenylene oxide (PPO). They also have good compatibility with these resins. The solubility as well as the volatility can be manipulated to some degree by variation of the various R groups. For example, the compounds have better solubility when there is a tert.-octyl group in the 5-position of the benzyl group instead of a methyl group. The bis-{N,N'-[2-hydroxy-3-(2H-benzotriazol-2-yl)-]oxamides} of the structure I where $R^3$ is a radical of Formula II are essentially non-volatile up to 300 °C.

The stabilizers of this invention can be used to stabilize polymeric compositions by merely mixing or blending with the polymeric composition, a compound of Formula I in an amount effective to stabilize the polymeric composition against the degradative effects of light (stabilizing effective amount).

The novel stabilizers of this invention can be blended with various polymeric compositions in high concentrations to form masterbatches which can then be blended with additional polymer either of the same or different type to form a light stabilized composition.

The stabilizing effective amount of stabilizer used to stabilize a particular polymeric composition will depend on the particular polymer system to be stabilized, the degree of stabilization desired and the presence of other stabilizers in the composition. One of skill in the art will readily be able to determine such an effective amount found on prior experience with analogous polymeric systems and knowledge of the properties of the compounds of the invention. Normally it is advisable to have about 0.01% to about 5% by weight of the UV absorber moiety of the compound of this invention present in the polymeric composition. An advantageous range is from about 0.05% to about 3% by weight of the UV absorber portion of the molecule in the final composition. In most cases about 0.1% to about 1% by weight is sufficient.

Non-limiting examples of polymeric compositions which may be stabilized by the stabilizer compounds of the present invention include:

1. Polyolefins, such as high, low and linear low density polyethylenes, which may be optionally crosslinked, polypropylene, polyisobutylene, poly(methylbutene-1), polyacetylene and, in general, polyolefins derived from monomers having from 2 to about 10 carbon atoms, and mixtures thereof.
2. Polyolefins derived from diolefins, such as polybutadiene and polyisoprene.
3. Copolymers of monoolefins or diolefins, such as ethylene-propylene, propylene-butene-1, propylene-isobutylene and ethylene-butene-1 copolymer.
4. Terpolymers of ethylene and propylene with dienes (EPDM), such as butadiene, hexadiene, dicyclopentadiene and ethylidene norbornene.
5. Copolymers of alpha-olefins with acrylic acid or methacrylic acids or their derivatives, such as ethylene-acrylic acid, ethylene-methacrylic acid and ethylene-ethyl acrylate copolymers.
6. Styrenic polymers, such as polystyrene (PS) and poly(p-methylstyrene).
7. Styrenic copolymers and terpolymers such as styrene-butadiene (SBR), styrene-allyl alcohol and styrene-acrylonitrile (SAN), styrene-acrylonitrile-methacrylate terpolymer, styrene-butadiene-styrene block copolymers (SBS), rubber modified styrenics such as styrene-acrylonitrile copolymers modified with acrylic ester polymer (ASA), graft copolymers of styrene on rubbers, such as polybutadiene (HIPS), polyisoprene or styrene-butadiene-styrene block copolymers (Stereon® products available from Firestone Synthetic Rubber and Latex Co.), graft copolymers of styrene-acrylonitrile on rubbers, such as butadiene (ABS), polyisoprene or styrene-butadiene-styrene block copolymers, graft copolymers of styrene-methyl methacrylate on rubbers, such as polybutadiene (MBS), butadiene-styrene radial block copolymers (e.g. KRO 3® of Phillips Petroleum Co.), selectively hydrogenated butadiene-styrene block copolymers (e.g. Kraton® G from Shell Chemical Co.), and mixtures thereof.
8. Polymers and copolymers derived from halogen-containing vinyl monomers, such as poly(vinyl chloride), poly(vinyl fluoride), poly(vinylidene chloride), poly(vinylidene fluoride), poly(tetrafluoroethylene) (PTFE), vinyl chloride-vinyl acetate copolymers, vinylidene chloride-vinyl acetate copolymers and ethylene-tetrafluoroethylene copolymers.
9. Halogenated rubbers, such as chlorinated and/or brominated butyl rubbers or polyolefins and fluoroelastomers.
10. Polymers and copolymers derived from alpha, beta-unsaturated acids, anhydrides, esters, amides and nitriles or combinations thereof, such as polymers or copolymers of acrylic and methacrylic acids, alkyl and/or glycidyl acrylates and methacrylates, acrylamide and methacrylamide, acrylonitrile, maleic anhydride, maleimide, the various anhydride containing polymers and copolymers described in this disclosure, copolymers of the polymers set forth in this paragraph and various blends and mixtures thereof, as well as rubber modified versions of the polymers and copolymers set forth in this paragraph.
11. Polymers and copolymers derived from unsaturated alcohols or their acylated derivatives, such as poly(vinyl alcohol), poly(vinyl acetate), poly(vinyl stearate), poly(vinyl benzoate), poly(vinyl maleate), poly(vinyl butyral), poly(allyl phthalate), poly(allyl diethylene glycol carbonate) (ADC), ethylene-vinyl acetate copolymer and ethylene-vinyl alcohol copolymers.
12. Polymers and copolymers derived from unsaturated amines, such as poly(allyl melamine).
13. Polymers and copolymers derived from epoxides, such as polyethylene oxide, polypropylene oxide and copolymers thereof, as well as polymers derived from bis-glycidyl ethers.
14. Poly(phenylene oxides), poly(phenylene ethers) and modifications thereof containing grafted polystyrene or rubbers, as well as their various blends with polystyrene, rubber modified polystyrenes or nylon.
15. Polycarbonates and especially the aromatic polycarbonates, such as those derived from phosgene and bisphenols such as bisphenol-A, tetrabromobisphenol-A and tetramethylbisphenol-A.
16. Polyesters derived from dicarboxylic acids and diols and/or hydroxycarboxylic acids or their corresponding lactones, such as polyalkylene phthalates (e.g. polyethylene terephthalate (PET), polybutylene terephthalate (PBT), and poly(1,4-dimethylcyclohexane terephthalate) or copolymers thereof) and polylactones such as polycaprolactone.
17. Polyarylates derived from bisphenols (e.g. bisphenol-A) and various aromatic acids, such as isophthalic and terephthalic acids or mixtures thereof.
18. Aromatic copolyestercarbonates having carbonate as well as ester linkages present in the backbone of the polymers, such as those derived from bisphenols, iso- and terephthaloyl chlorides and phosgene.
19. Polyurethanes and polyureas.
20. Polyacetals, such as polyoxymethylenes and polyoxymethylenes which contain ethylene oxide as a comonomer.
21. Polysulfones, polyethersulfones and polyimidesulfones.
22. Polyamides and copolyamides which are derived from diamines and dicarboxylic acids and/or from aminocarboxylic acids or the corresponding lactones, such as the following nylons: 6, 6/6, 6/10, 11 and

12.

23. Polyimides, polyetherimides, polyamideimides and copolyetheresters.

24. Cross-linked polymers which are derived from aldehydes on the one hand and from phenols, ureas and melamine on the other hand, such as phenol-formaldehyde, urea-formaldehyde and melamine-formaldehyde resins.

25. Alkyd resins, such as glycerol-phthalic acid resins and mixtures thereof with melamine-formaldehyde resins.

26. Blends of vinyl monomers and unsaturated polyester resins which are derived from copolyesters of saturated and unsaturated dicarboxylic acids with polyhydric alcohols, as well as from vinyl compounds (crosslinking agents) and also halogen-containing, flame resistant modifications thereof.

27. Natural polymers, such as cellulose and natural rubber, as well as the chemically modified homologous derivatives thereof, such as cellulose acetates, cellulose propionate, cellulose butyrate and the cellulose ethers, such as methyl and ethyl cellulose.

In addition, the novel stabilizers of this invention may be used to stabilize various combinations or blends of the above polymers or copolymers. They are particularly useful in the stabilization of polyolefins, acrylic coatings, styrenics, rubber modified styrenics, poly(phenylene oxides) and their various blends with styrenics, rubber-modified styrenics, nylon and especially polycarbonate and its various blends with ABS. The novel ultraviolet light absorbers of this invention can be used together with other additives to further enhance the properties of the finished polymer. Examples of other additives that can be used in conjunction with the stabilizers of this invention include antioxidants, such as alkylated monophenols, alkylated hydroquinones, hydroxylated thiodiphenyl ethers, alkylidene-bis-phenols, hindered phenolic benzyl compounds, acylaminophenols, esters of 3-(3,5-di-t-butyl-4-hydroxyphenyl)propionic acid, esters of 3-(5-t-butyl-4-hydroxy-3-methylphenyl)propionic acid, 3-(3,5-di-t-butyl-4-hydroxyphenyl)propionic acid amides; other UV absorbers and light stabilizers such as 2-hydroxybenzophenones, benzylidene malonate esters, esters of substituted or unsubstituted benzoic acids, diphenyl acrylates, nickel chelates, oxalic acid diamides, and hindered amine light stabilizers; other additives such as metal deactivators, phosphites and phosphonites, peroxide decomposers, fillers and reinforcing agents, plasticizers, lubricants, corrosion and rust inhibitors, emulsifiers, mold release agents, carbon black, pigments, fluorescent brighteners, both organic and inorganic flame retardants and nondripping agents, melt flow improvers and antistatic agents. Numerous examples of suitable additives of the above type are given in Canadian Patent 1,190,038.

## Preparative Methods

The novel N-[2-hydroxy-3-(2H-benzotriazol-2-yl)benzyl]oxamides of Formula I can be prepared by several methods.

## METHOD 1.

Method 1 applies for compounds of Formula I where $R^2$ is hydrogen and $R^3$ is not an aryl group. It also applies for compounds of Formula I wherein $R^3$ is a radical of Formula II

where X is the diradical

$$-NH-\overset{\overset{O}{\|}}{C}-\overset{\overset{O}{\|}}{C}-\overset{\overset{R^2}{|}}{N}-R^4-$$

and

where $R^2$ is hydrogen and $R^4$ is not an aryl diradical. Preferably R is hydrogen, chlorine or alkyl of 1-8 carbons and $R^1$ is alkyl of 1-8 carbons or 1-methyl-1-phenylethyl.

Aminomethyl-substituted benzotriazoles of Formula IV are reacted with alkyl oxalyl chlorides in the presence of an acid acceptor to prepare N-[2-hydroxy-3-(2H-benzotriazol-2-yl)benzyl]oxamate esters of Formula V.

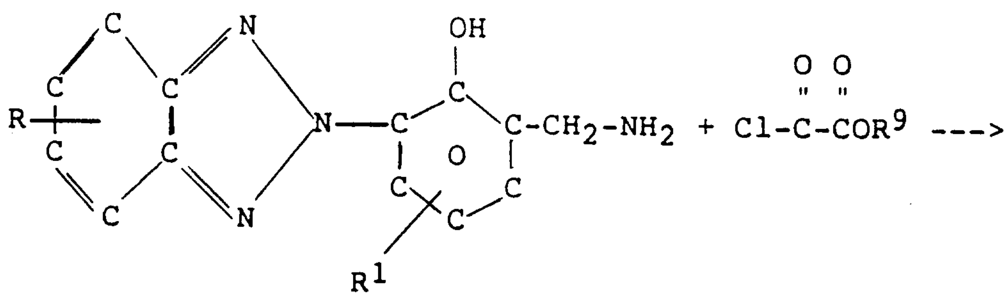

IV

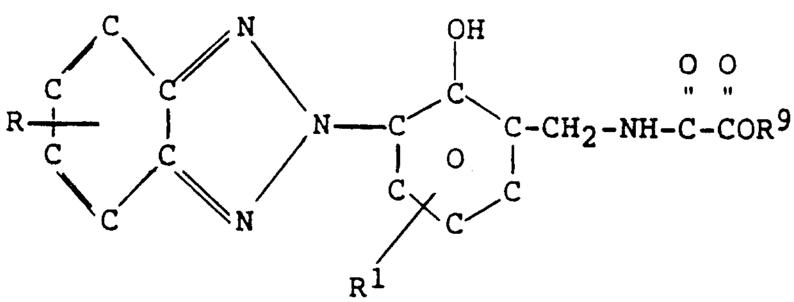

V

$R^9$ is lower alkyl of 1-4 carbons or allyl. The acid acceptor is preferably a tertiary amine such as triethylamine or pyridine or an excess of the aminomethyl-benzotriazole (IV). The reaction is preferably run in an inert solvent such as a hydrocarbon, chlorinated hydrocarbon or ether and the temperature is preferably held between 0-35°C during the reaction. The mole ratio of the alkyl oxalyl chloride to IV is approximately 1:1 if a tertiary amine acid acceptor is used.

Similar esters Va may be prepared by reaction of the aminomethyl-benzotriazole (IV) with lower dialkyl oxalates or diphenyl oxalate.

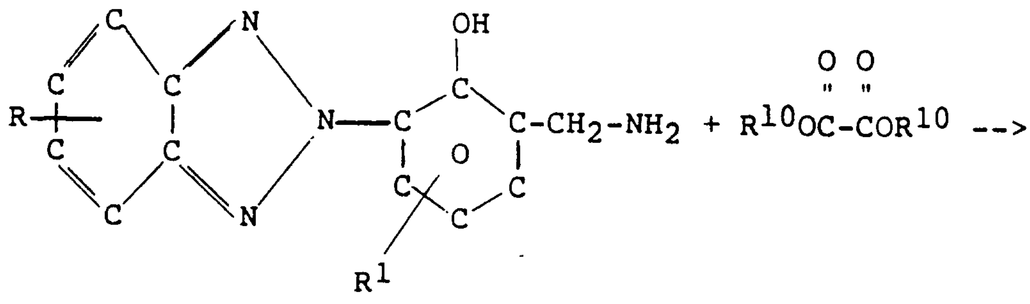

IV

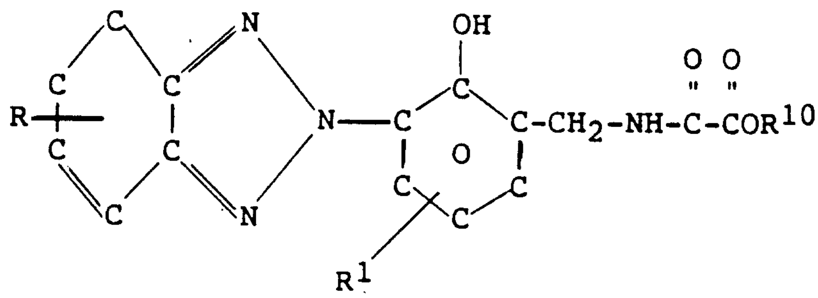

Va

$R^{10}$ is lower alkyl of 1-4 carbons or phenyl. The mole ratio of the oxalate to the aminomethyl-benzotriazole is 1:1 or greater. If an excess of the oxalate is used it can be stripped from the product at the end of the reaction or it can be washed out of the product with a solvent in which the product is insoluble. Preferably, the reaction is run neat or in an inert polar solvent such as methanol, ethanol, propanol, isopropanol, diethyl ether, tetrahydrofuran or methyl tert.-butyl ether below 40°C. Most preferably, the reaction is run in methanol at room temperature.

The preparation of the aminomethyl-benzotriazoles (IV) are described in U.S. Patents 3,862,087 and 3,629,192.

The oxamate esters of Formula V or Va are reacted with primary aliphatic, cycloaliphatic, heterocyclic or polyoxyalkylene amines to form the Formula I compounds, where $R^2$ is hydrogen and $R^3$ is not an aryl group.

$$V \text{ (or Va)} \quad + \quad R^3NH_2 \quad \text{------>}$$

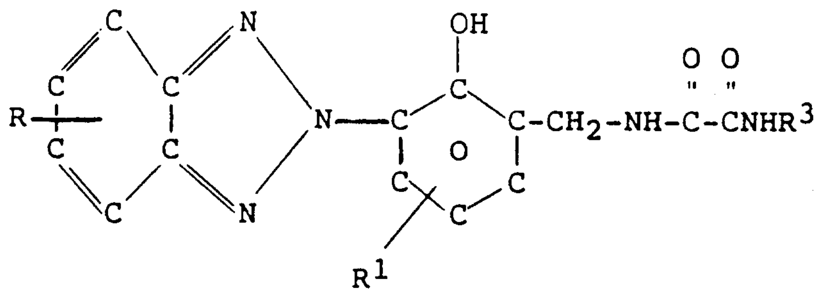

The oxamate esters of Formula V or Va are reacted with primary aliphatic, cycloaliphatic, araliphatic or polyoxyalkylene diamines to form the compounds of Formula I wherein $R^3$ is a radical of Formula II wherein X is a diradical of the formula

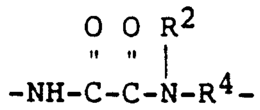

and where $R^2$ is hydrogen and $R^4$ is not an aryl diradical.

V (or Va) + $H_2N-R^4-NH_2$ ------>

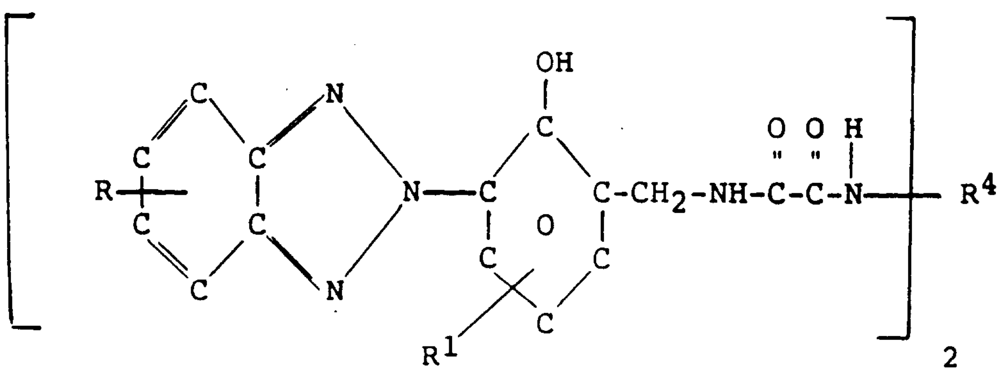

If the mole ratio of V (or Va) to diamine is approximately 2:1, the major product is compound I wherein $R^3$ is a radical of Formula II as illustrated immediately above. If the mole ratio of V (or Va) to diamine is less than 2:1, then some of the compound of Formula I where $R^3$ is $-R^4-NH_2$ will also form.

$$V \text{ (or Va)} + xs\ H_2N\text{-}R^4\text{-}NH_2 \text{ ------>}$$

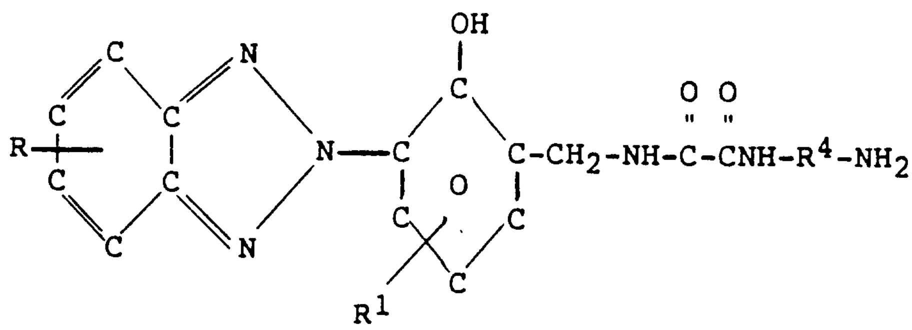

The oxamate esters of Formula V (or Va) are reacted with the same or different aminomethyl-substituted benzotriazole of Formula IV to form compounds of Formula I where $R^3$ is a radical of Formula II and $R^2$ is hydrogen.

V (or Va) + IV ------> I

The reaction may be run in an inert polar solvent at or below the reflux temperature of the solvent. Preferably, the reaction is run in refluxing methanol, ethanol, propanol or isopropanol. Most preferably, the reaction is run in refluxing methanol. The disappearance of the oxamate ester(V or Va) as the reaction progresses can be followed by liquid chromatography.

The oxamate esters of Formula V (or Va) are reacted with 4-amino-substituted-2,2,6,6-tetramethylpiperidines to form compounds of Formula I where $R^3$ is a radical of Formula III.

V (or Va) +

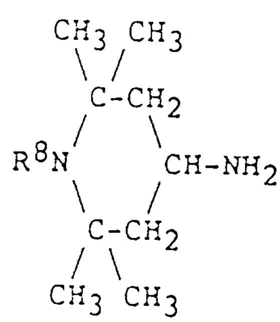

------> I

where $R^2$ is hydrogen and $R^3$ is a radical of Formula III. Preferably, the reaction is carried out in methanol. (Preferably R = H, $R^1$ = methyl, $R^{10}$ = methyl or ethyl, $R^8$ = H, the solvent is methanol, ethanol, propanol or isopropanol, the mole ratio of the two compounds is 1:1 and the reaction is carried out between 20 °C and the reflux temperature of the solvent.)

<u>METHOD 2</u>

This method applies for all the novel compounds of Formula I.

Lower alkyl oxalyl chlorides are reacted with primary or secondary amines ($R^2R^3NH$ where $R^2$ and $R^3$ are as previously defined) in an inert solvent in the presence of an acid acceptor to form intermediates of Formula VI.

$$R^9O\overset{O}{\overset{\|}{C}}-\overset{O}{\overset{\|}{C}}-Cl \quad + \quad R^2R^3NH \quad \text{----->} \quad R^9O\overset{O}{\overset{\|}{C}}-\overset{O}{\overset{\|}{C}}-NR^2R^3$$

VI

Intermediates VIa may be prepared by reacting lower alkyl oxalates or diphenyl oxalate with primary or secondary amines in approximately a 1:1 mole ratio [A. G. Richardson, J. Scanton Pierce and E. Emmet Reid, J. Am. Chem. Soc., 71,4011-12(1952), R. J. Cremyln, J. Chem. and Eng. Data, 19(3), 288-94(1974), Y. A. Naumov, A. N. Mentus, M. V. Goryacheva and A. N. Kost, Zh. Organ. Khimii 12 (12), 2507-2511(1976)].

$$R^{10}O\overset{O}{\overset{\|}{C}}-\overset{O}{\overset{\|}{C}}OR^{10} \quad + \quad R^2R^3NH \quad \text{---->} \quad R^{10}O\overset{O}{\overset{\|}{C}}-\overset{O}{\overset{\|}{C}}-NR^2R^3$$

VIa

With the aromatic amines it is often preferable to run the reaction neat in the presence of boric acid and distil off the alcohol as it forms [U.S.Patent 3,683,020]. Suitable solvents for the reaction are higher boiling solvents such as xylene and diphenyl ether.

The intermediates of Formula VI or VIa are reacted with aminomethyl-substituted benzotriazoles of Formula IV to form the novel compounds of Formula I.

IV + VI (or VIa) ----> I + $R^9OH$ (or $R^{10}OH$)

The reaction may be carried out in a polar solvent at or below the reflux temperature of the solvent using approximately a 1:1 mole ratio of IV to VI (or VIa). Preferably, the reaction is carried out in refluxing methanol, ethanol, propanol or isopropanol. Most preferably, the reaction is carried out in refluxing methanol. The extent of reaction can be monitored by liquid chromatography.

Lower alkyl oxalyl chlorides are reacted with primary or secondary diamines [$HN(R^2)-R^4-N(R^2)H$ where $R^2$ and $R^4$ are as previously defined] in an inert solvent in the presence of an acid acceptor to form intermediates of Formula VII.

$$2 \quad R^9O\overset{O}{\overset{\|}{C}}-\overset{O}{\overset{\|}{C}}-Cl \quad + \quad HN(R^2)-R^4-N(R^2)H \quad \text{----->}$$

$$R^9O\overset{O}{\overset{\|}{C}}-\overset{O}{\overset{\|}{C}}-N(R^2)-R^4-N(R^2)-\overset{O}{\overset{\|}{C}}-\overset{O}{\overset{\|}{C}}OR^9$$

VII

The acid acceptor is preferably a tertiary amine and the reaction is preferably run in an inert solvent such as a hydrocarbon, chlorinated hydrocarbon or ether solvent and the reaction temperature is held between -5 °C and 35 °C during the reaction. The mole ratio of the lower alkyl oxalyl chloride to the diamine is preferably about 2:1 or slightly greater.

The intermediate VIIa may be prepared by reacting lower alkyl oxalates or diphenyl oxalate with primary or secondary diamines $HN(R^2)-R^4-N(R^2)H$ in approximately a 2-3:1 mole ratio of the oxalate to the

diamine.

O O
" "
2 $R^{10}OC-COR^{10}$ + $HN(R^2)-R^4-N(R^2)H$ ----->

O O O O
" " " "
$R^{10}OC-C-N(R^2)-R^4-N(R^2)-C-COR^{10}$

VIIa

With the aromatic diamines it is often preferable to run the reaction neat or in the presence of a solvent boiling above 120 °C in the presence of a small amount of boric acid and distil off the alcohol or phenol as it forms (U.S.Patent 3,683,020).

The intermediates of Formula VII or VIIa are reacted with aminomethyl-substituted benzotriazoles of Formula IV to form the novel compounds of Formula I wherein $R^3$ is a radical of Formula II and X is

O O $R^2$
" " |
$-NH-C-C-N-R^4-$

O O $R^2$
" " |
2 IV + VII (or VIIa) ----> I ($R^3$ = II, X = $-NHC-C-N-R^4-$)
+ $R^9OH$ (or $R^{10}OH$)

The reaction is carried out in a polar solvent, at or below the reflux temperature of the solvent using approximately a 2:1 ratio of IV to the intermediate VII or VIIa. Preferably, the reaction is carried out in refluxing methanol, ethanol, propanol or isopropanol. Most preferably, the reaction is carried out in refluxing methanol. The extent of reaction can be monitored by liquid chromatography.

METHOD 3

This method is applicable for the preparation of N,N'-di-[2-hydroxy-3-(2H-benzotriazol-2-yl)benzyl]-oxamides. N,N'-bis(hydroxymethyl)oxamide is prepared from excess formalin, oxamide and a catalytic amount of potassium bicarbonate according to the method of Gilbert [E.Gilbert, J. Heterocyclic Chemistry, 8,327-8(1971)].

The N,N'-bis(hydroxymethyl)oxamide is reacted with a solution of a 2-(2-hydroxyphenyl-2H-benzotriazole of Formula VIII to form the N,N'-bis-[2-hydroxy-3-(2H-benzotriazol-2-yl)benzyl]oxamides of Formula I where $R^2$ is hydrogen and $R^3$ is a radical of Formula II wherein X is a direct bond and $R^5$ is the same as R and $R^6$ is the same as $R^1$.

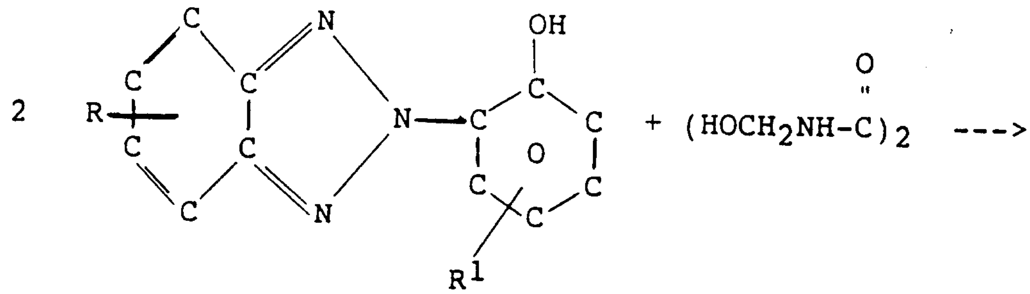

VIII

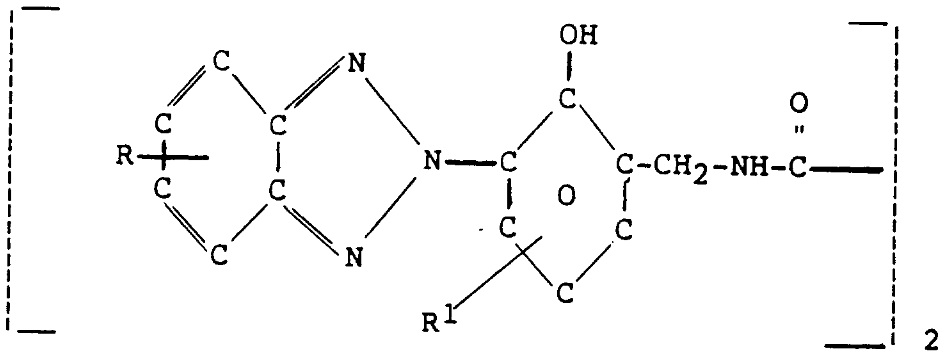

The reaction is run in concentrated sulfuric acid (90-100%) at -5 to 15 °C for 1-3 hours. The mole ratio of VIII to N,N'-bis(hydroxymethyl)oxamide is approximately 2:1. The product is preferably isolated by pouring the concentrated acid solution over ice and filtering off the solids that form. The solids may be washed to a pH of 5-6 and dried. Small amounts of impurities and residual benzotriazole VIII can be removed by slurrying the crude product in solvents, preferably hot, such as methanol, ethanol, isopropanol, toluene, hexane, or ethyl acetate. The product is only slightly soluble in the hot solvents and is isolated in a purified state by separating the insoluble product from the hot solvent by filtration.

Non-limiting examples of suitable oxalyl halides for reaction with aminomethyl-substituted benzotriazoles(IV) or primary or secondary aliphatic, cycloaliphatic, araliphatic, aromatic heterocyclic or polyoxyalkylene amines include methyl oxalyl chloride, ethyl oxalyl chloride, propyl oxalyl chloride and isopropyl oxalyl chloride.

Non-limiting examples of suitable oxalates for reaction with aminomethyl-substituted benzotriazoles(IV) or primary or secondary amines ($R^2R^3NH$) or diamines [$HN(R^2)-R^4-N(R^2)H$] where $R^2$, $R^3$ and $R^4$ are as previously defined, include dimethyl oxalate, diethyl oxalate, dipropyl oxalate, diisopropyl oxalate, dibutyl oxalate, diphenyl oxalate and methyl ethyl oxalate.

The following examples of 2-(2'-hydroxyphenyl)-2H-benzotriazoles are suitable for conversion to 2-(2'-hydroxy-3-aminomethyl-phenyl)-2H-benzotriazoles by reaction with a hydroxymethylamide followed by saponification (see U.S.Patent 3,629,192). These same 2-(2'-hydroxyphenyl)-2H-benzotriazoles are also suitable for reaction with N,N'-bis(hydroxymethyl)oxamide via Method 3 to form the N,N'-bis[2-hydroxy-3-(2H-benzotriazol-2-yl)benzyl]oxamides.

The non-limiting list includes:

2-(2'-hydroxy-5'-methylphenyl)-2H-benzotriazole,
2-(2'-hydroxy-5'-methylphenyl)-2H-5-chlorobenzotriazole,
2-(2'-hydroxy-5'-tert.-butylphenyl)-2H-benzotriazole,
2-(2'-hydroxy-5'-tert.-butylphenyl)-2H-5-chlorobenzotriazole,
2-(2'-hydroxy-5'-tert.-octylphenyl)-2H-benzotriazole,
2-(2'-hydroxy-4'-octoxyphenyl)-2H-benzotriazole,
2-(2'-hydroxy-5'-cyclohexylphenyl)-2H-benzotriazole,
2-(2'-hydroxy-5'-amylphenyl)-2H-benzotriazole,

2-(2'-hydroxy-5'-methoxyphenyl)-2H-benzotriazole,
2-(2'-hydroxy-5'-methylphenyl)-2H-5-ethylbenzotriazole,
2-(2'-hydroxy-5'-benzylphenyl)-2H-benzotriazole,
2-(2'-hydroxy-5'-phenylphenyl)-2H-benzotriazole,
2-(2'-hydroxy-5'-methylphenyl)-2H-5-bromobenzotriazole,
2-(2'-hydroxy-5'-methylphenyl)-2H-5-carboxybenzotriazole,
2-(2'-hydroxy-5'-phenylphenyl)-2H-4-chlorobenzotriazole,
2-(2'-hydroxy-5'-butoxyphenyl)-2H-benzotriazole,
2-(2'-hydroxy-5'-cyclopentylphenyl)-2H-benzotriazole,
2-(2'-hydroxy-5'-chlorophenyl)-2H-benzotriazole,
2-(2'-hydroxy-5'-bromophenyl)-2H-benzotriazole.

Non-limiting examples of suitable aminomethyl-substituted benzotriazoles for reaction with alkyl oxalyl chlorides, alkyl oxalates, diphenyl oxalate and N-[2-hydroxy-3-(2H-benzotriazol-2-yl)benzyl]oxamate esters include:

2-(2'-hydroxy-3'-aminomethyl-5'-methylphenyl)-2H-benzotriazole,
2-(2'-hydroxy-3'-aminomethyl-5'-methylphenyl)-5-chloro-2H-benzotriazole,
2-(2'-hydroxy-3'-aminomethyl-5'-tert.-butylphenyl)-2H-benzotriazole,
2-(2'-hydroxy-3'-aminomethyl-5'-tert.-butylphenyl)-2H-5-chlorobenzotriazole,
2-(2'-hydroxy-3'-aminomethyl-5'-tert.-octylphenyl)-2H-benzotriazole,
2-(2'-hydroxy-3'-aminomethyl-4'-octoxyphenyl)-2H-benzotriazole,
2-(2'-hydroxy-3'-aminomethyl-5'-cyclohexylphenyl)-2H-benzotriazole,
2-(2'-hydroxy-3'-aminomethyl-5'-amylphenyl)-2H-benzotriazole,
2-(2'-hydroxy-3'-aminomethyl-5'-methoxyphenyl)-2H-benzotriazole,
2-(2'-hydroxy-3'-aminomethyl-5'-methylphenyl)-2H-5-ethylbenzotriazole,
2-(2'-hydroxy-3'-aminomethyl-5'-benzylphenyl)-2H-benzotriazole,
2-(2'-hydroxy-3'-aminomethyl-5'-phenylphenyl)-2H-benzotriazole,
2-(2'-hydroxy-3'-aminomethyl-5'-methylphenyl)-2H-5-bromobenzotriazole,
2-(2'-hydroxy-3'-aminomethyl-5'-methylphenyl)-2H-5-carboxybenzotriazole,
2-(2'-hydroxy-3'-aminomethyl-5'-phenylphenyl)-2H-5-chlorobenzotriazole,
2-(2'-hydroxy-3'-aminomethyl-5'-butoxyphenyl)-2H-benzotriazole,
2-(2'-hydroxy-3'-aminomethyl-5'-cyclopentylphenyl)-2H-benzotriazole,
2-(2'-hydroxy-3'-aminomethyl-5'-chlorophenyl)-2H-benzotriazole,
2-(2'-hydroxy-3'-aminomethyl-5'-bromophenyl)-2H-benzotriazole.

Non-limiting examples of suitable primary aliphatic, cycloaliphatic, araliphatic, heterocyclic or polyoxyalkylene amines for reaction with the N-[2-hydroxy-3-(2H-benzotriazol-2-yl)benzyl]oxamate esters of Formula V or Va in Method 1 or with the oxalyl chlorides or the oxalates in Method 2 to form the intermediates VI or VIa include anhydrous ammonia, methyl, ethyl, propyl, isopropyl, n-butyl, isobutyl, sec.-butyl, tert.-butyl, n-amyl, isoamyl, sec.-amyl, tert.-amyl, n-hexyl, 1,3-dimethylbutyl, n-heptyl, sec.-heptyl, allyl, methallyl, n-octyl, tert.-octyl, n-decyl, n-dodecyl, n-hexadecyl, n-octadecyl, 2-ethylhexyl, cyclohexyl, cyclopentyl and benzyl amines, 2-amino-2-methyl-1-propanol, 1-amino-2-propanol, 5-hydroxy-3-oxapentylamine, 3-hydroxypropylamine, 3-aminopentanol, 3-amino-2,2-dimethylpropanol, 2-amino-2-methyl-4-hydroxypentane, ethanolamine, tris(hydroxymethyl)aminomethane, 2-aminoethoxyethanol, mono isopropanolamine, 2-methyl-6-phenyl-4-hexylamine, cyclopropylamine, 12-aminododecanoic acid, 2-(aminoethyl)pyridine, 2-aminoheptane, 1-aminoindane, 2-bromobenzylamine, cycloheptylamine, cyclododecylamine, 1,5-dimethylhexylamine, 1,1-dimethylpropargylamine, 2-ethoxybenzylamine, 3-methoxybenzylamine, 4-methylcyclohexylamine, oleylamine, 4-amino-2,2,6,6-tetramethylpiperidine, 4-amino-1,2,2,6,6-pentamethylpiperidine, 4-amino-1-allyl-2,2,6,6-tetramethylpiperidine, 4-amino-1-acetyl-2,2,6,6-tetramethylpiperidine, 4-amino-1-benzoyl-2,2,6,6-tetramethylpiperidine, 4-amino-1-hydroxyethyl-2,2,6,6-tetramethylpiperidine, 4-amino-1-benzyl-2,2,6,6-tetramethylpiperidine, 6-amino-2-mercaptobenzothiazole, 5-aminobenzotriazole, 1-(m-aminophenyl)-5-mercaptotetrazole and Jeffamines® M-600, M-1000, M-2005 and M-2070 (products of Texaco Chemical Co.) which are monoamino terminated polyethers where the polyether backbone is based on propylene oxide, ethylene oxide or a combination thereof.

Non-limiting examples of suitable primary aliphatic, cycloaliphatic, araliphatic or polyoxyalkylene diamines for reaction with the N-[2-hydroxy-3-(2H-benzotriazol-2-yl)benzyl]oxamate esters of Formula V or Va in Method 1 or with the oxalyl chlorides or the oxalates in Method 2 to form the intermediates VII or VIIa include ethylenediamine, propylenediamine, tetramethylenediamine, 2-methylpentamethylenediamine, 2-ethylhexamethylenediamine, hexamethylenediamine, octamethylenediamine, nonamethylenediamine, dodecamethylenediamine, 2,5-dimethyl-2,5,-hexanediamine, 1,3-diamino-2-hydroxypropane, bis(p-aminocyclohexyl)methane and Jeffamines® D-230, D-400, D-2000, D-4000, ED-600, ED-900, ED-2001, ED-4000 (products of Texaco Chemical Co.) which are diamino terminated polyethers where the polyether backbone is based on propylene oxide, ethylene oxide or a combination thereof.

Non-limiting examples of suitable primary diamines containg hetero atoms in the alkylene chain for reaction with the N-[2-hydroxy-3-(2H-benzotriazol-2-yl)benzyl]oxamate esters of structure V or Va in Method 1 or with the oxalyl chlorides or the oxalates in Method 2 to form the intermediates VII or VIIa include diethylenetriamine, triethylenetetramine, tetraethylenepentamine, pentaethylenehexamine, N-methyldipropylenetriamine, N-aminopropyl-ethylenediamine, N,N'-bis(aminopropyl)ethylenediamine and 1,4-bis(3-aminopropoxy)butane

Non-limiting examples of suitable primary amino substituted heterocycles for reaction with the N-[2-hydroxy-3-(2H-benzotriazol-2-yl)benzyl]oxamate esters of Formula V or Va in Method 1 or with the oxalyl chlorides or the oxalates in Method 2 to form the intermediates VI or VIa include 2-aminobenzimidazole, 2-amino-5,6-dimethylbenzothiazole, 2-amino-4,5-dimethylthiazole, 5-amino-3-methylisothiazole, 3-amino-5-methylisoxazole and 2-aminobenzothiazole.

Non-limiting examples of suitable primary or secondary amines of structure $R^2R^3NH$ for reaction with lower alkyl oxalyl chlorides, lower aklyl oxalates or diphenyl oxalate to form intermediates VI or VIa in Method 2 include diethanolamine, diethylamine, dimethylamine, diallylamine, diisopropylamine, diisopropanolamine, dibutylamine, diisobutylamine, dioctylamine, dicyclohexylamine, dibenzylamine, N-butylbenzylamine, morpholine, diisooctylamine, piperidine, 2, 3, or 4-pyridylamines, aniline, o, m, or p-toluidine, o, m or p-methoxyaniline, o, m, or p-chloroaniline, 2,4-dichloroaniline, 3,4-dichloroaniline, o-anisidine, o, m or p-aminophenol, 2,4-, 2,5-, 2,6- and 3,5-xylidene, tert.-butylaniline, 2-chloro-4-toluidine, m-amino-p-cresol, 4-chloro-2-aminophenol, o, m and p-anisidine, p-cresidine, o and p-phenetidine, ethylcresidine, p-aminophenylglycol, o, m and p-aminobenzoic acid methyl and ethyl esters, anthranilic acid methyl and ethyl esters, alpha-naphthylamine, 1-amino-7-naphthol, 2-aminobiphenyl, 4-aminobiphenyl, N-methylaniline, N-ethylaniline, N-methyl-o-toluidine, N-ethyl-o-toluidine, N-methyl-m-toluidine, N-ethyl-p-toluidine, N-n-butylaniline, N-hydroxyethylaniline and N-ethyl-alpha-naphthylamine.

Additional non-limiting examples of suitable primary or secondary diamines for reaction with the lower alkyl oxalyl chlorides or the oxalates in Method 2 to form the intermediates VII or VIIa include o, m and p-phenylenediamine, o, m and p-toluylenediamine, 2,4-diaminoanisole, 3,4-diaminobenzoic acid methyl and ethyl esters, 1,5-naphthylenediamine and 1,8-naphthylenediamine.

Non-limiting examples of suitable polar solvents for the reactions of intermediates V, Va, VI or VIa with amines or diamines in preparative methods 1 or 2 include diethyl ether, methyl tert.-butyl ether, dimethylformamide, dimethylacetamide, tetrahydrofuran, dioxane, pyrrolidone, N-methylpyrrolidone, acetone, methyl ethyl ketone and methyl isobutyl ketone.

## EXPERIMENTAL

The following examples are presented to provide a more detailed explanation of the present invention and are intended as illustrations and not limitations of the invention.

## STARTING MATERIALS

Tinuvin® P, [2-(2'-hydroxy-5'-methylphenyl)benzotriazole], was obtained from Ciba-Geigy Corporation.

Cyasorb® 5411, [2-(2'-hydroxy-5'-tert.-octylphenyl)benzotriazole], was obtained from American Cyanamid Company.

Aminomethyl Tinuvin® P,
[2-(2'-hydroxy-3'-aminomethyl-5'-methylphenyl)benzotriazole], was obtained by the saponification of 2-(2'-hydroxy-3'-acetamidomethyl-5'-methylphenyl)benzotriazole] (see U.S. Patent 3,629,192).

Jeffamines® M-360, D-230 and D-400 were obtained from the Texaco Chemical Company.

N,N'-bis(hydroxymethyl)oxamide was prepared according to the method of Gilbert [E. Gilbert, J. Heterocyclic Chemistry, 8, 327-8 (1971)]. A 4:1 mole ratio of formalin to oxamide was used.

Ethyl N-(2-methoxyphenyl)oxamate was prepared in 96% yield from o-anisidine and ethyl oxalyl chloride. The melting point was 75-78 °C.

Ethyl N-(2-ethoxyphenyl)oxamate was prepared in 82% yield from o-phenetidine and ethyl oxalyl chloride. The melting point was 67-69 °C.

Ethyl N-(4-hydroxyphenyl)oxamate was prepared in 72% yield from 4-aminophenol and ethyl oxalyl chloride. The melting point was 173-178 °C.

Ethyl N-phenyloxamate was prepared in 95% yield from aniline and diethyl oxalate using the method of Pierce [J.S. Pierce et.al., J. Amer. Chem. Soc., 73, 2595 (1951)]. The melting point was 59-61 °C.

Calibre® 300-6 polycarbonate was obtained from Dow Chemical U.S.A.

Mixxim® BB/100, bis[2-hydroxy-5-tert.-octyl-3-(benzotriazol-2-yl)phenyl]methane, was obtained from Fairmount Chemical Company.

EXAMPLE I

Preparation of Ethyl N-[2-hydroxy-3-(2H-benzotriazol-2-yl)-5-methylbenzyl]oxamate

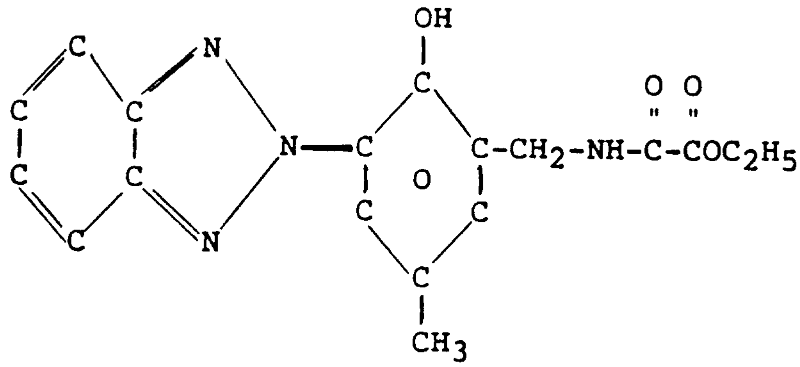

Into a 3-liter, 3-neck round bottom flask was added 35.2 grams (0.24 mole) diethyl oxalate and 1000 mls of methanol. The flask was equipped with a mechanical stirrer, thermometer and a reflux condenser. To the room temperature solution was added 62.1 grams (0.22 mole) of aminomethyl Tinuvin® P in small portions with vigorous stirring over 15 minutes. The reaction mixture was stirred for an additional 4 hours at room temperature. Liquid chromatography scans indicated the reaction was complete after 2 hours. The solids were filtered off, washed with 200 mls of fresh methanol and air dried. The product weighed 67.2 grams (86.3 % yield) and had a melting point of 167-170 °C. The infrared scan of the product contained strong sharp carbonyls at 1730 and 1675 $cm^{-1}$.

An additional 2.8 grams of product were obtained by concentrating the methanol filtrate and filtering off the solids that formed.

Examples II-XIV

Preparation of N-[2-hydroxy-3-(2H-benzotriazol-2-yl)-5-methylbenzyl]-N'-alkyloxamides

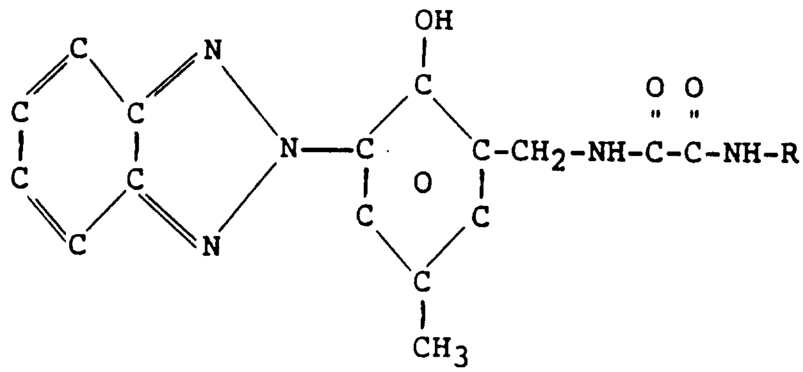

General Procedure

Into a 300 ml 3-neck flask was added 10.6 grams (0.03 mole) ethyl N-[2-hydroxy-3-(2H-benzotriazol-2-yl)-5-methylbenzyl]oxamate (from Example I) and 100-200 mls of methanol. The flask was equipped with a magnetic stirrer, thermometer, reflux condenser and a dropping funnel containing 0.032 mole of the appropriate amine in 15 mls of methanol. The amine solution was added dropwise to the stirring benzotriazole solution. Upon addition of the amine, there was a slight exotherm and the reaction mixture thickened up. The reaction mixture was stirred 1/2 hour at room temperature and then heated in an oil bath to reflux and refluxed approximately 2 hours. The hot reaction mixture was filtered and the insoluble product was washed with fresh methanol and air dried. Melting points, infrared scans and thermogravimetric scans were run on the dry products. The results are summarized in Table I. Ultraviolet scans were run on some of the compounds. The wavelengths of the maximum absorption for the two UV absorption peaks ($max_1$ and $max_2$) and the molar (or equivalent) absorptions ($e_1$ and $e_2$) at those wavelengths for the selected compounds are found in Table IV.

The product in Example XIII was soluble in methanol and was isolated by filtering off any insolubles and then stripping off the methanol from the filtrate on a rotating evaporator under reduced pressure. The product was a viscous liquid.

Example XV

Preparation of N-[2-hydroxy-3-(2H-benzotriazol-2-yl)-5-methylbenzyl]-N'-(6-aminohexyl)oxamide

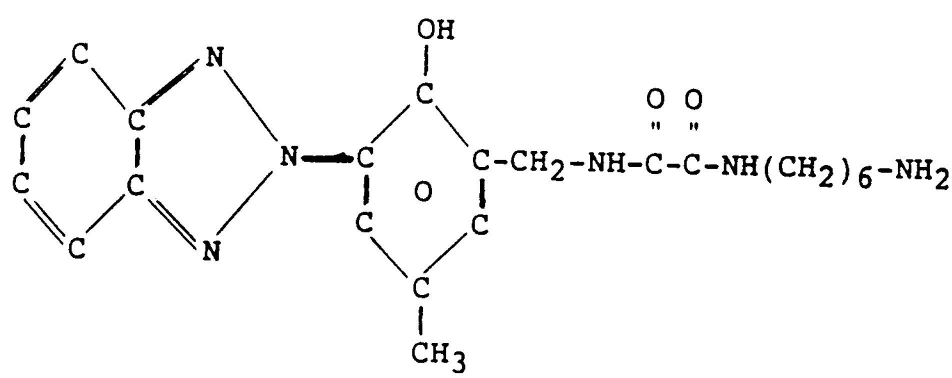

The above product was prepared using the general procedure of Examples II-XIV except a large excess of 1,6-hexanediamine was used, the addition sequence was reversed and the addition was carried out at

55°C to insure only one amine group of the diamine would react with the ethyl N-[2-hydroxy-3-(2H-benzotriazol-2-yl)-5-methylbenzyl]oxamate.

Into a 300 ml 3-neck flask was added 11.6 grams (0.1 mole) of 1,6-hexanediamine and 200 mls of methanol. The flask was equipped with a magnetic stirrer, thermometer and reflux condenser. The solution was heated to 55 °C and 10.6 grams (0.3 mole) of ethyl N-[2-hydroxy-3-(2H-benzotriazol-2-yl)-5-methylbenzyl]oxamate added in small portions over 15 minutes. After the addition was complete, the reaction was heated to reflux and refluxed 2 hours. The hot methanol slurry was filtered. The insoluble product was slurried in 250 mls of acetone and refiltered. The filter cake was reslurried in 300 mls of fresh methanol and refiltered. After air drying, the filter cake weighed 9.0 gram (93% yield). The product had a melting point of 210-220 °C. An infrared scan of the product had a strong sharp carbonyl peak at 1650 $cm^{-1}$ and a medium sharp carbonyl peak at 1510 $cm^{-1}$.

Examples XVI-XIX

Preparation of N,N'-alkylene(or alkyleneoxyalkylene)-bis{N'-[2-hydroxy-3-(2H-benzotriazol-2-yl)-5-methylbenzyl]oxamides}

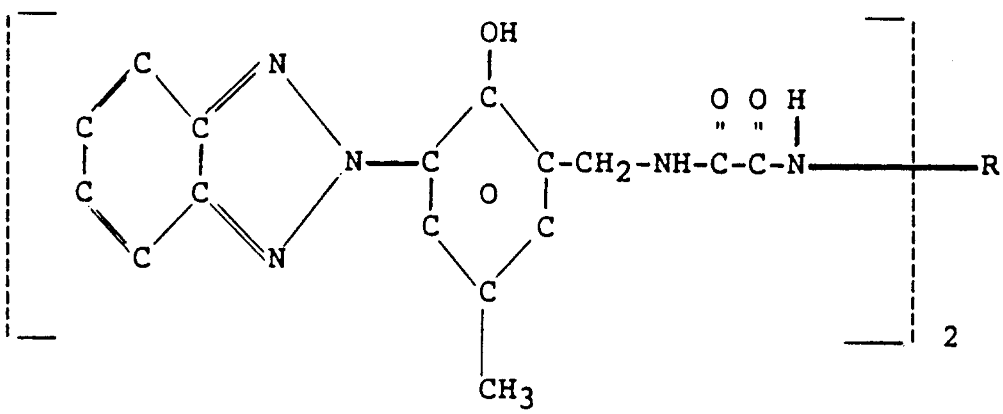

<u>General Procedure</u>

Into a 300 ml 3-neck flask was added 10.6 gms (.03 mole) ethyl N-[2-hydroxy-3-(2H-benzotriazol-2-yl)-5-methylbenzyl]oxamate (from Example I) and 200 mls of methanol. The flask was equipped with a magnetic stirrer, thermometer, reflux condenser and a dropping funnel containing .015 mole of the appropriate diamine in 15 mls of methanol. The diamine solution was added dropwise to the stirring benzotriazole solution. Upon addition of the diamine, there was a slight exotherm and the reaction mixture thickened up. The reaction mixture was stirred 1/2 hour at room temperature and then heated in an oil bath to reflux and refluxed approximately 2 hours. The hot reaction mixture was filtered and the insoluble product was washed with fresh methanol and air dried. Melting points and infrared scans were run on the dry products. Thermogravimetric scans were run on Examples XVI and XVII and the onset of weight loss determined. The results are summarized in Table II.

Example XX

Preparation of N-[2-hydroxy-3-(2H-benzotriazol-2-yl)-5-methylbenzyl]-N'-tert.-amyloxamide

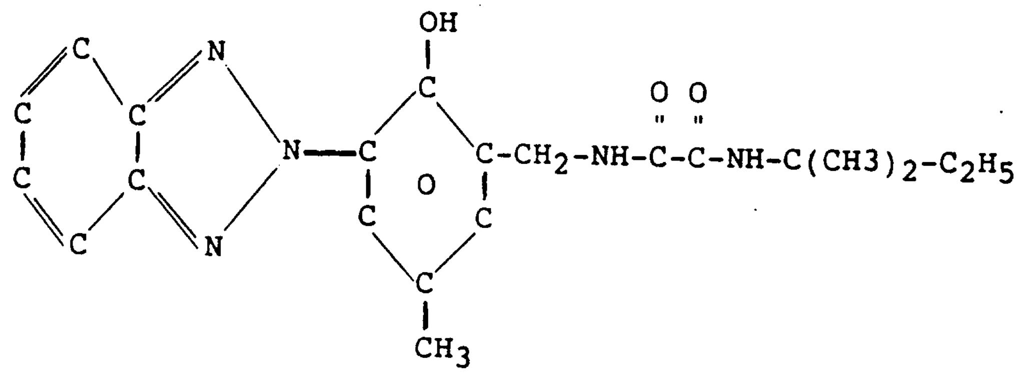

A) Preparation of Ethyl N-tert.-amyloxamate

Into a 250 ml 3-neck flask was added 17.4 grams (0.2 moles) of tert.-amylamine and 100 mls of methylene chloride. The flask was equipped with a thermometer, magnetic stirrer, reflux condenser and dropping funnel containing 13.7 grams (0.1 mole) ethyl oxalyl chloride. The ethyl oxalyl chloride was added dropwise to the stirring tert.-amylamine solution over 15 minutes, allowing the temperature to rise from 22 to 39 ° C where refluxing began to occur. The reaction was allowed to stir overnight at room temperature. The next day the reaction mixture was diluted with 100 mls of water, transferred to a separatory funnel and the methylene chloride layer separated. The methylene chloride layer was washed with another 100 mls of water, dried over anhydrous sodium sulfate and the methylene chloride stripped off on a rotating evaporator under reduced pressure. The residue was a clear liquid weighing 15.8 gms (84.5% yield). An infrared scan of the product contained a strong sharp carbonyl band at 1700 $cm^{-1}$ with a weak shoulder at 1730 $cm^{-1}$. It also contained a medium sharp carbonyl band at 1520 $cm^{-1}$.

B) Reaction of Ethyl N-tert.-amyloxamate with Aminomethyl Tinuvin® P

Into a 250 ml 3-neck flask was added 7.5 grams (.04 mole) of ethyl Ntert.-amyloxamate and 150 mls of methanol. The flask was equipped with a thermometer, reflux condenser and magnetic stirrer. To the stirring solution was added 12.0 gms (.04 mole) of approximately 85% aminomethyl Tinuvin® P in small portions over 8-10 minutes. The reaction mixture was stirred for 20 minutes at room temperature and then heated to reflux and refluxed for 2 hours. The hot reaction mixture was filtered hot. The insolubles were slurried in 150 mls of fresh methanol and heated back up to reflux and refiltered. The insolubles had a melting point of 260-275 ° C and were discarded. Solids crystallized out of the hot methanol filtrates upon cooling. They were filtered off and air dried. The combined crops weighed 9.9 gms (62.6% yield) and had a melting point of 109-113 ° C. The infrared scan of the product contained a strong sharp carbonyl band at 1665 $cm^{-1}$ and a medium sharp carbonyl band at 1510 $cm^{-1}$.

Example XXI

Preparation of N-[2-hydroxy-3-(2H-benzotriazol-2-yl)-5-methylbenzyl]-N'-tert.-octyloxamide

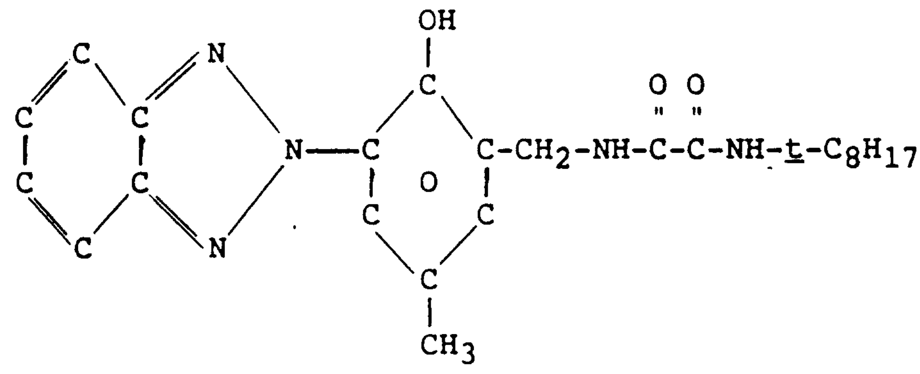

A) Preparation of Ethyl N-tert.-octyloxamate

Ethyl N-tert.-octyloxamate was prepared from ethyl oxalyl chloride and tert.-octylamine using the procedure of Example XX A. The product, a straw colored liquid, was obtained in 93.4% yield. The infrared scan contained a strong carbonyl at 1700 $cm^{-1}$ and a weaker carbonyl band at 1520 $cm^{-1}$. A liquid chromatograph scan showed one sharp peak.

B) Reaction of N-tert.-octyloxamate with Aminomethyl Tinuvin® P

Aminomethyl Tinuvin® P (.04 mole) was reacted with N-tert.-octyloxamate (.04 mole) using the procedure of Example XX B. The product was isolated by crystallizing it out of hot methanol. The yield was 7.2 gms (41% yield) and the product had a melting point of 120-123 °C. The infrared scan of the product contained a strong carbonyl band at 1660 $cm^{-1}$ and a weaker carbonyl at 1515 $cm^{-1}$.

Example XXII

Preparation of N-[2-hydroxy-3-(2H-benzotriazol-2-yl)-5-methylbenzyl]oxamide

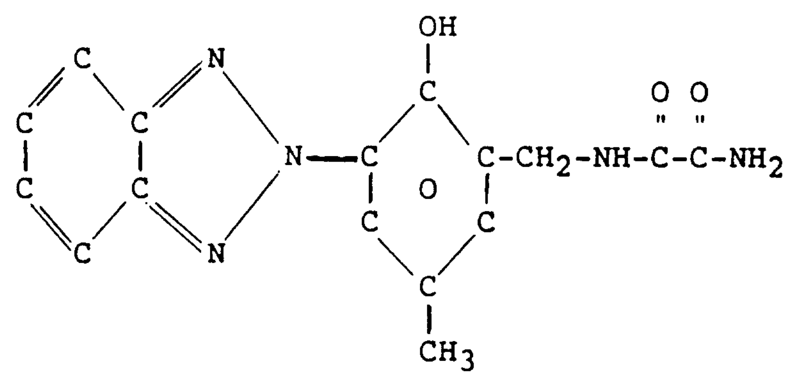

Into a 300 ml 3-neck flask was added 6.7 gms (.055 mole) ethyl oxamate and 200 mls of methanol. The flask was equipped with thermometer, reflux condenser and magnetic stirrer. To the stirring solution was added 12.7 gms (.05 mole) of aminomethyl Tinuvin® P in small portions over 5 minutes. The reaction was then heated to reflux and refluxed for 2 1/2 hours. The reaction mixture was filtered hot. The filter cake was slurried in 200 mls of acetone and refiltered. The product weighed 12.0 gms (74% yield) after air drying and had a melting point of 275-285 °C. The infrared scan of the product contained a strong carbonyl band at

1655 $cm^{-1}$ and a moderate carbonyl band at 1540 $cm^{-1}$.

Examples XXIII-XXVI

Preparation of N-[2-hydroxy-3-(2H-benzotriazol-2-yl)-5-methylbenzyl]-N'-aryloxamides

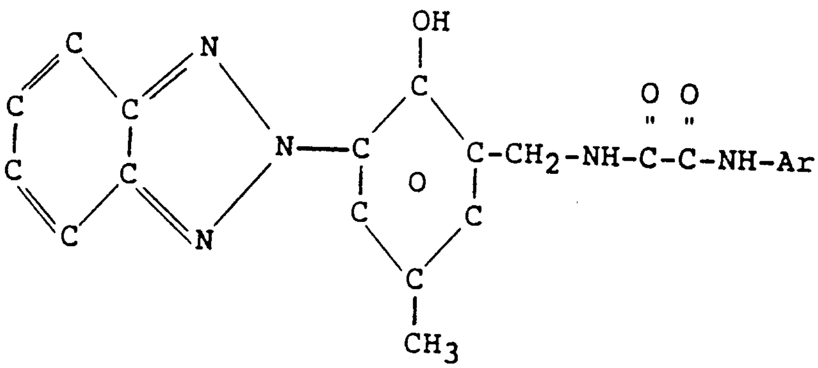

Into a 300 ml 3-neck flask was added .04 moles of the appropriate ethyl N-aryloxamate and 150-200 mls of methanol. The flask was equipped with a thermometer, reflux condenser and magnetic stirrer. To the stirring solution was added .04 moles aminomethyl Tinuvin® P in small portions over 5-10 minutes. The reaction mixture was then heated to reflux, refluxed for 2 hours and filtered hot. The insoluble product was washed with acetone and air dried. Melting points and infrared scans were run on the dry products. Thermogravimetric scans were run on Examples XXIII and XXVI and the onset of weight loss determined. The results are summarized in Table III. Ultraviolet scans were run on the compounds and the results are summarized in Table IV. There is a considerable enhancement of the UV absorption at 300 nanometers for these aryl substituted compounds. The molar absorptions are compared to the molar absorptions of commercial products. It should be noted that Mixxim® BB/100 is difunctional and therefore its equivalent absorption is used.

Example XXVII

Preparation of N,N'-bis[2-hydroxy-3-(2H-benzotriazol-2-yl)-5-(tert.-octyl)benzyl]oxamide

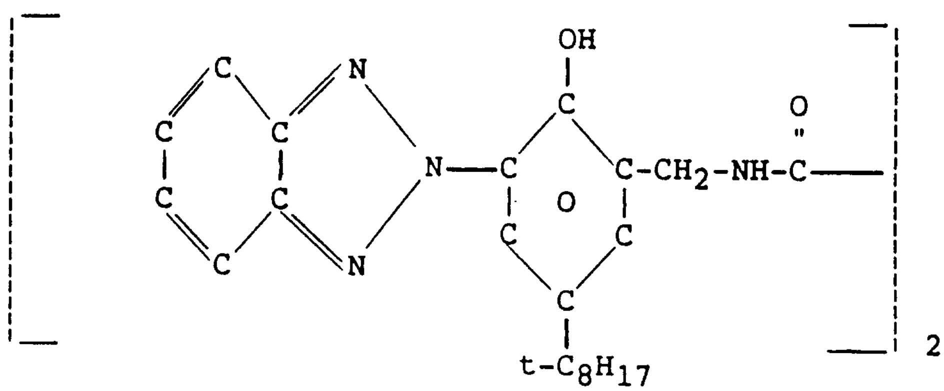

Into a 1 liter 3-neck flask was added 400 mls of concentrated sulfuric acid (96%). The flask was equipped with a mechanical stirrer, thermometer and condenser. The stirrer was activated and 103.6 gms (0.32 mole) of Cyasorb® 5411 was added to the stirring sulfuric acid over 5 minutes at 25°C. The mixture was stirred 20 minutes at room temperature to allow the Cyasorb® 5411 to dissovle completely in the acid. The solution was then cooled to 0 ° C in an ice-salt bath and 26.2 gms (0.168 mole) of N,N'-bis-

(hydroxymethyl)oxamide was added in small portions over 30 minutes while holding the reaction temperature between 0 °C and -2 °C. Upon completion of the addition, the reaction mixture was stirred an additional 2 hours at 0 °C and then stirred into a mixture of 1200 gms of ice and 2000 mls of deionized water. The crude product precipitated out of solution and was filtered off. The filter cake was washed to pH of 6 with deionized water and air dried over the weekend. The crude product was purified by slurrying it with hot ethyl acetate for 15 minutes and filtering it hot. The filter cake was air dried, crushed and vacuum dried at 50 °C. The recovered product weighed 100 grams (78% yield) and had a melting point of 204-210 °C.

The infrared spectrum of the product contained a strong carbonyl group at 1680 $cm^{-1}$ with a shoulder at 1655 $cm^{-1}$ and a strong carbonyl at 1506 $cm^{-1}$. The ultraviolet scan was run in tetrahydrofuran. The results are summarized in Table IV. The equivalent absorbtion is given since the product is difunctional. A thermo- gravimetric analysis indicated the onset of weight loss at 364 °C.

EXAMPLE XXVIII

Preparation of N,N'-bis[2-hydroxy-3-(2H-benzotriazol-2-yl)-5-methylbenzyl]oxamide

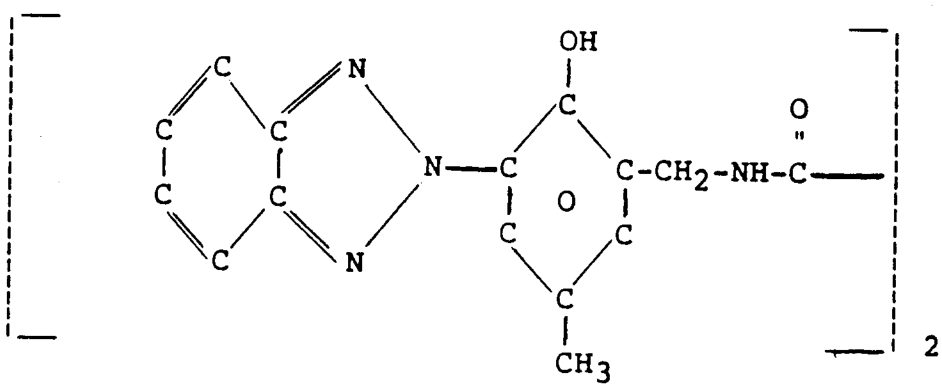

Into a 250 ml 3-neck flask was added 100 mls of concentrated sulfuric acid (96%). The flask was equipped with a magnetic stirrer, thermometer and condenser. The stirrer was activated and 18.7 grams (.083 mole) of Tinuvin® P was addded over 5 minutes at 25 °C. The mixture was stirred about 20 minutes at room temperature to allow the Tinuvin® P to dissolve completely in the acid. The solution was then cooled to 0 °C in an ice-salt bath and 6.9 grams (.042 mole) of 93% N,N'-bis(hydroxymethyl)oxamide was added in small portions over 20 minutes while holding the reaction temperature between 0 °C and -2 °C. The reaction mixture was stirred for 1 hour at 0 °C, the ice bath was removed and the reaction stirred an additional 2 hours while warming up to room temperature. The reaction mixture was then poured over 300 grams of ice and diluted with 100 mls of deionized water. The product precipitated out of solution and was filtered off. The filter cake was washed to a pH of 6 with deionized water and dried overnight. The crude product was purified by slurrying it in 125 mls of refluxing methanol for 15 minutes and then filtered hot. The filter cake was air dried overnight. The product weighed 23.1 grams (78% yield). The product had a melting point of 290-294 °C and its infrared spectrum was identical to that of another sample prepared by an alternate route in Example XIV. The product was not soluble enough to run an ultraviolet scan in THF.

EXAMPLES XXIX-XXXVII

Preparation, Weathering and Evaluation of Tensile Bars Containing N-[2-hydroxy-3-(2H-benzotriazol-2-yl)benzyl]oxamides

Polycarbonate blends were prepared by vacuum drying 598.2 grams of Calibre® 300-6 polycarbonate in a vacuum oven at 120 °C for 4 hours and then mixing the hot polycarbonate pellets with 0.6 gms of Primal 355 T light mineral oil and then with 1.8 gms (0.3%) of the UV absorber. The blend was transferred to a jar and tightly sealed. The jar was shaken well to insure uniform distribution of the mineral oil and UV absorber on the polycarbonate. The blends were extruded on a Brabender Prep Center Extruder Model No. 1340 having a 1 1/4 inch screw diameter with a length to diameter ratio of 25:1. The extruder was operated

at a screw speed of 25 RPM and all the heating zones were controlled at 280 °C. The first 100 grams of extrudate were used to purge out the extruder between runs and was discarded. The remaining extrudate was air-cooled and pelletized.

The pellets were redried for 1 hour at 120°C in a vacuum oven and injected molded in a Newbury 25 ton injection molding machine at 530°F into 7-3/8" x 3/4" x 1/8" tensile bars.

Control samples with and without Cyasorb® 5411 were prepared in the same manner for comparison.

The initial YID values of the tensile bars were determined on a Colorgard System 105 (Pacific Scientific) colorimeter. Then the tensile bars were placed in a QUV accelerated Weathering Tester (Q Panel Company). The QUV operated with an 8 hour light cycle (UV-A bulbs) at 60 °C and a 4 hour condensation cycle at 50 °C. The tensile bars were periodically withdrawn from the QUV and the YIDs determined on the colorimeter and the samples placed back in the QUV. The tensile bars bleached during the first week of exposure and then gradually began to yellow. The polycarbonate control without any UV absorber began yellowing immediately and the YID increased rapidly to a value of 50 over 45 days. In contrast, the samples containing our novel UV absorbers increased in YID to a value of 20-30 over 45 days and to a value of 25-35 over 108 days. The delta YID values were in the range of 11-19 compared to 49 for the control. The final YID values and the delta YID values are summarized in Table V. The control containing Cyasorb® 5411 gave comparable results to samples containing our novel UV absorbers. However, Cyasorb® 5411 is much more volatile than the novel compounds of this invention, having an onset of weight loss temperature of about 260 °C compared to temperatures of 310 to 385°C for the compounds of this invention.

Table I

N-[2-hydroxy-3-(2H-benzotriazol-2-yl)-5-methylbenzyl]-N'-alkyloxamides

| EXAMPLE # | AMINE RNH2 | GRAMS AMINE | YIELD | % YIELD | M.P. (°C) | TGA ONSET OF WEIGHT LOSS (°C) | IR CARBONYL BANDS ($CM^{-1}$) |
|---|---|---|---|---|---|---|---|
| II | DECYLAMINE | 5.05 | 12.25 | 88 | 181-190 | 350 | 1660(s) 1530(s) 1515(w) |
| III | OCTYLAMINE | 4.15 | 11.7 | 89 | 188-195 | 334 | 1660(s) 1530(s) 1510(w) |
| IV | CYCLOHEXYLAMINE | 3.2 | 9.1 | 75 | 240-252 | 356 | 1650(s) 1520(m) |
| V | DIETHYLAMINO-PROPYLAMINE | 4.2 | 11.9 | 91 | 159-166 | 345 | 1652(s) 1520(m) |
| VI | 3-AMINOPROPYL-TRIETHOXYSILANE | 7.1 | 13.2 | 83 | 163-168 | 320 | 1655(s) 1520(m) 1510(w) |
| VII | DIMETHYLAMINO-PROPYLAMINE | 3.3 | 10.8 | 88 | 183-189 | 309 | 1655(s) 1525(m) 1510(w) |
| VIII | ALLYLAMINE | 1.85 | 9.6 | 88 | 206-214 | 308 | 1655(s) 1520(m) |
| IX | BUTYLAMINE | 2.4 | 10.1 | 88 | 218-224 | 331 | 1650(s) 1510(m) |
| X | OCTADECYLAMINE | 8.6 | 16.1 | 93 | 173-178 | 366 | 1655(s) 1520(m) |
| XI | ETHANOLAMINE | 1.83 | 10.5 | 95 | 251-259 | | 1650(s) 1520(m) |
| XII | 4-AMINO-2,2,6,6-TETRAMETHYLPIPERIDINE | 4.8 | 9.9 | 71 | 180-184 | | 1650(s) 1500(s) |
| XIII | JEFFAMINE® M-360 | 11.9 | 14.5 | 73 | VISC LIQ | | 1670(s) 1655(s) 1500(m) |
| XIV | AMINOMETHYL-TINUVIN® P | 7.7 | 14.0 | 79 | 290-294 | 360 | 1655(s) 1510(w) |

Table II

N-N'-Alkylenebis{N'-[2-hydroxy-3-(2H-benzotriazol-2-yl)-5-methylbenzyl]oxamides}

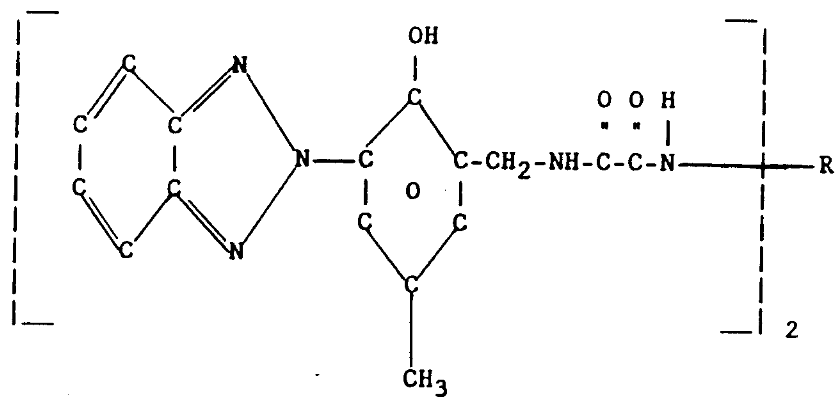

| EXAMPLE # | DIAMINES | GRAMS DIAMINE | YIELD | % YIELD | M.P.(°C) | TGA ONSET OF WEIGHT LOSS (°C) | IR CARBONYL BANDS ($CM^{-1}$) |
|---|---|---|---|---|---|---|---|
| XVI | 1,6-HEXANE-DIAMINE | 1.7 | 10.2 | 99 | 275-285 | 373 | 1650(s) 1510(s) |
| XVII | ETHYLENE-DIAMINE | 0.9 | 9.7 | 96 | > 300 | 384 | 1655(s) 1520(w) |
| XVIII | JEFFAMINE® D-400 | 6.0 | 5.4 | 35 | 245-247 | | 1655(m) 1515(w) |
| XIX | JEFFAMINE® D-230 | 3.45 | 7.5 | 59 | 180-200 | | 1650(s) 1515(m) |

Table III

N-[2-HYDROXY-3-(2H-BENZOTRIAZOL-2-yl)-5-METHYLBENZYL]-N'ARYLOXAMIDES

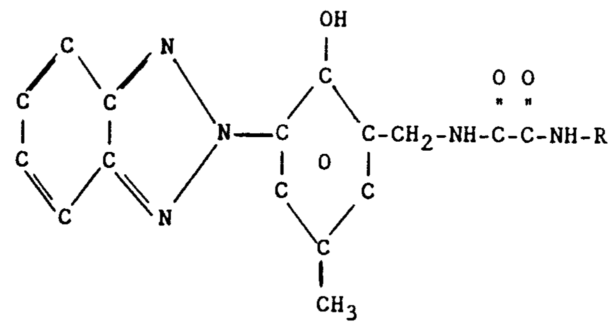

| EXAMPLE # | STARTING N-ARYL OXAMATE | GRAMS OXAMATE | YIELD OXAMIDE | % YIELD | M.P.(°C) | TGA ONSET OF WEIGHT LOSS (°C) | IR CARBONYL BANDS ($CM^{-1}$) |
|---|---|---|---|---|---|---|---|
| XXIII | ETHYL N-(2-METHOXY-PHENYL)-OXAMATE | 8.9 | 13.0 | 75 | 217-224 | 345 | 1670(s) 1600(w) 1525(m) |
| XXIV | ETHYL N-(2-ETHOXY-PHENYL)-OXAMATE | 9.5 | 11.2 | 63 | 194-204 | | 1665(s) 1600(w) 1530(m) |
| XXV | ETHYL N-PHENYLOXAMATE | 7.7 | 11.8 | 73 | 260-265 | | 1665(s) 1600 (w) 1520(m) |
| XXVI | ETHYL N-(4-HYDROXY-PHENYL)-OXAMATE | 8.4 | 12.0 | 65 | 270-275 | 340 | 1650(s) 1610(w) 1510(m) |

Table IV

ULTRAVIOLET ABSORPTION IN THF

| EXAMPLE | EQ. WT. | MAX(1) | e(1) | MAX(2) | e(2) |
|---|---|---|---|---|---|
| II | 465 | 300 | 18,000 | 338 | 19,650 |
| III | 437 | 300 | 18,150 | 338 | 19,900 |
| X | 578 | 300 | 16,200 | 338 | 18,000 |
| XIII | 431 | 298 | 24,400 | 338 | 20,150 |
| XIV | 445 | 300 | 24,800 | 339 | 20,105 |
| XV | 400 | 297 | 20,000 | 339 | 18,950 |
| XVI | 417 | 297 | 24,600 | 338 | 20,000 |
| XXVII | 380 | 298 | 15,700 | 338 | 16,825 |
| CYASORB(R) 5411 | 325 | 298 | 14,400 | 341 | 15,900 |
| TINUVIN(R) P | 225 | 300 | 14,400 | 342 | 16,950 |
| MIXXIM(R) BB100 | 329 | 303 | 15,900 | 344 | 16,400 |

e(1) and e(2) are the molar extinction coefficients at the wavelengths of maximum absorption divided by the number of benzotriazole groups per molecule.

TABLE V

QUV-A STABILIZATION OF POLYCARBONATE WITH

N-[2-hydroxy-3-(benzotriazol-2-yl)benzyl]oxamides

| EXAMPLE # | UV ABSORBER | INITIAL YID | DAYS QUV-A EXPOSURE | FINAL YID | DELTA YID |
|---|---|---|---|---|---|
| XXIX | III | 18.8 | 60 | 29.0 | 10.2 |
| XXX | IX | 11.5 | 108 | 30.7 | 19.2 |
| XXXI | X | 23.0 | 87 | 37.0 | 14.0 |
| XXXII | XVI | 18.1 | 60 | 34.1 | 16.0 |
| XXXIII | XXII | 19.7 | 108 | 35.0 | 15.3 |
| XXXIV | XXIII | 24.7 | 108 | 35.6 | 11.0 |
| XXXV | XXVI | 20.5 | 108 | 35.2 | 14.7 |
| XXXVI | XXVII | 10.9 | 108 | 29.6 | 18.8 |
| XXXVII | XXVIII | 13.8 | 108 | 29.0 | 15.2 |
| CONTROL | CYASORB(R) 5411 | 10.0 | 60 | 24.4 | 14.4 |
| CONTROL | CYASORB(R) 5411 | 10.0 | 108 | 26.0 | 16.0 |
| CONTROL | NONE | 12.8 | 60 | 57.4 | 44.6 |
| CONTROL | NONE | 12.8 | 87 | 59.3 | 46.5 |
| CONTROL | NONE | 12.8 | 108 | 62.2 | 49.4 |

## Claims

**1.** Oxamides having the Formula I:

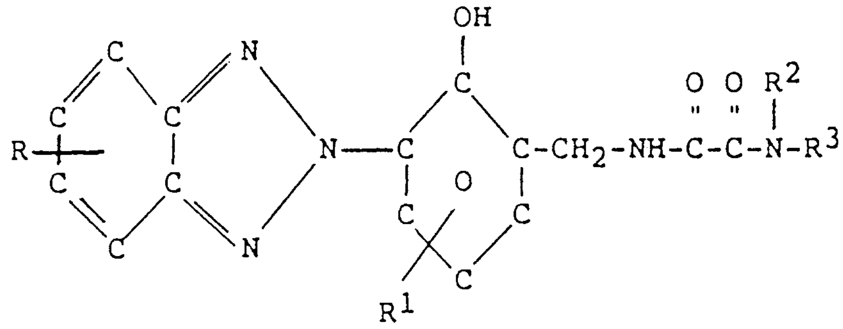

I

wherein:

R is selected from hydrogen, chlorine, bromine, substituted or unsubstituted aliphatic of 1-8 carbons, substituted or unsubstituted alkoxy of 1-8 carbons, substituted or unsubstituted alkoxycarbonyl of 2-8 carbons, alkylaminocarbonyl of 2-5 carbons, dialkylaminocarbonyl of 3-9 carbons, substituted or unsubstituted N-(alkyl)-N-(aryl)aminocarbonyl of 8-15 carbons, alkoxysulfonyl of 1-4 carbons, -C(=O)-OH, -C(=O)$NH_2$, or -S(=O)$_2$-OH;

$R^1$ is selected from hydrogen, chlorine, bromine, substituted or unsubstituted aliphatic of 1-8 carbons, substituted or unsubstituted aryl of 6-14 carbons, substituted or unsubstituted araliphatic of 7-22 carbons, substituted or unsubstituted alicyclic of 5-12 carbons, substituted or unsubstituted alkoxy of 1-8 carbons, alkylaminocarbonyl of 2-5 carbons, dialkylaminocarbonyl of 3-9 carbons, or substituted or unsubstituted N-(alkyl)-N-(aryl)aminocarbonyl of 8-15 carbons,

$R^2$ is selected from hydrogen, substituted or unsubstituted aliphatic of 1-20 carbons, substituted or unsubstituted araliphatic of 7-22 carbons, or substituted or unsubstituted alicyclic of 5-12 carbons,

$R^3$ is selected from hydrogen, substituted or unsubstituted aliphatic of 1-20 carbons, substituted or unsubstituted alicyclic of 5-12 carbons, substituted or unsubstituted araliphatic of 7-22 carbons, substituted or unsubstituted aryl of 6-14 carbons, substituted or unsubstituted aromatic or non-aromatic heterocyclic group of 5-12 carbons where the heteroatom may be oxygen, nitrogen or sulfur, polyoxyalkylene of about 9-44 alkyleneoxide linkages or a radical of Formula II or III

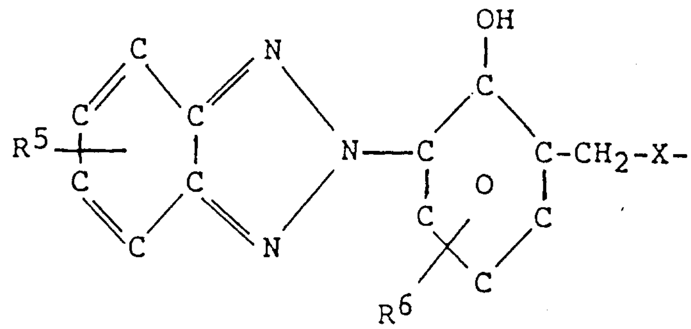

II

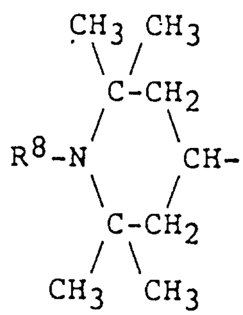

III

wherein X is a direct bond or the diradical:

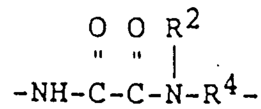

wherein NH is bonded to the methylene of II and $R^4$ is bonded to the oximino nitrogen of I;

$R^2$ and $R^3$ may be concatenated to form a substituted or unsubstituted heterocyclic ring including the nitrogen atom to which they are attached of 5-12 carbons or may be concatenated through -N($R^7$)-, -O-, and -S- to form a heterocyclic ring including the nitrogen to which they are attached of 6-12 atoms wherein heteroatoms are separated by at least two carbon atoms;

$R^4$ is selected from substituted or unsubstituted aliphatic diradicals of 1-20 carbons, substituted or unsubstituted aryl diradicals of 6-12 carbons, substituted or unsubstituted alicyclic diradicals of 5-12 carbons, or substituted or unsubstituted araliphatic diradicals of 7-22 carbons, the aliphatic chains of which may optionally contain -O-, -S- and -N($R^7$)- with the proviso that multiple heteroatoms must be separated from each other and the diradical ends by at least two carbon atoms or polyoxyalkylene diradicals of about 3-34 alkyleneoxide linkages;

$R^5$ is selected from hydrogen, chlorine, bromine, substituted or unsubstituted aliphatic of 1-8 carbons, substituted or unsubstituted alkoxy of 1-8 carbons, substituted or unsubstituted alkoxycarbonyl of 2-8 carbons, -C(=O)-OH, -C(=O)$NH_2$, or -S(=O)$_2$-OH;

$R^6$ is selected from hydrogen, substituted or unsubstituted aliphatic of 1-8 carbons, substituted or unsubstituted aryl of 6-14 carbons, substituted or unsubstituted araliphatic of 7-22 carbons, substituted or unsubstituted alkoxy of 1-8 carbons;

$R^7$ is selected from hydrogen, substituted or unsubstituted aliphatic of 1-20 carbons, substituted or

unsubstituted alicyclic of 5-12 carbons, substituted or unsubstituted araliphatic of 7-22 carbons, substituted or unsubstituted aliphatic acyl of 2-20 carbons, substituted or unsubstituted alicyclic acyl of 7-16 carbons, substituted or unsubstituted araliphatic acyl of 8-23 carbons or substituted or unsubstituted aryl acyl of 7-11 carbons;

$R^8$ is selected from hydrogen, oxyl, alkyl of 1-8 carbons, alkenyl or alkynyl of 3-12 carbons, aliphatic acyl of 1-12 carbons, aromatic acyl of 7-15 carbons, aralkyl of 7-9 carbons or hydroxyalkyl of 2-6 carbons, and optional substituents for R, $R^1$, $R^2$, $R^3$, $R^4$, $R^5$, $R^6$ and $R^7$ are independently selected from one or more of the following: amino, alkylamino of 1-4 carbons, dialkylamino of 2-8 carbons, chloro, bromo, alkyl of 1-8 carbons, alkoxy of 1-8 carbons, phenoxy, cyano, hydroxy, epoxy, carboxy, alkoxycarbonyl of 2-6 carbons, acryloyl, methacryloyl, acyloxy of 1-6 carbons, acryloyloxy, methacryloyloxy, hydroxymethyl, hydroxyethyl, alkylthio of 1-4 carbons, or trialkoxysilyl of 3-12 carbons.

**2.** Oxamides as defined in claim 1 wherein:

R is selected from hydrogen, substituted or unsubstituted aliphatic of 1-8 carbons, substituted or unsubstituted alkoxy of 1-8 carbons, methoxycarbonyl, ethoxycarbonyl, carboxy, chloro or $S(=O)_2$-OH;

$R^1$ is selected from hydrogen, substituted or unsubstituted aliphatic of 1-8 carbons, or substituted or unsubstituted araliphatic of 7-9 carbons;

$R^2$ is selected from hydrogen or lower alkyl of 1-6 carbons;

$R^3$ is selected from hydrogen, substituted or unsubstituted aliphatic of 1-18 carbons, substituted or unsubstituted alicyclic of 6-10 carbons, substituted or unsubstituted araliphatic of 7-15 carbons, substituted or unsubstituted aryl of 6-14 carbons, polyoxyalkylene of about 9-44 alkyleneoxide linkages or a radical of Formula II or III;

$R^2$ and $R^3$ may be concatenated to form together with the nitrogen atom to which they are attached a substituted or unsubstituted piperidine, piperazine or morpholine ring;

$R^4$ is selected from substituted or unsubstituted aliphatic diradicals of 2-12 carbons, 1,3- or 1,4-phenylene diradicals or polyoxyalkylene diradicals of about 3-34 alkyleneoxide linkages;

$R^5$ is selected from hydrogen, substituted or unsubstituted aliphatic of 1-8 carbons, substituted or unsubstituted alkoxy of 1-8 carbons, methoxycarbonyl, ethoxycarbonyl, carboxy, chloro or $S(=O)_2$-OH;

$R^6$ is selected from hydrogen, substituted or unsubstituted aliphatic of 1-8 carbons, or substituted or unsubstituted araliphatic of 7-9 carbons;

$R^7$ is selected from hydrogen, substituted or unsubstituted araliphatic of 7-10 carbons, substituted or unsubstituted aliphatic acyl of 2-6 carbons or substituted or unsubstituted benzoyl; and

$R^8$ is selected from hydrogen, alkyl of 1-4 carbons, alkenyl of 3 or 4 carbons, benzyl, acetyl or benzoyl.

**3.** Oxamides as defined in claim 2 wherein:

R is selected from hydrogen, alkyl of 1-4 carbons, methoxy, ethoxy, chloro, or carboxy, $R^1$ is methyl, ethyl, tert.-butyl, tert.-octyl, or 1-methyl-1-phenylethyl;

$R^2$ is hydrogen;

$R^3$ is selected from hydrogen, substituted or unsubstituted aliphatic of 3-18 carbons, substituted or unsubstituted alicyclic of 6-8 carbons, substituted or unsubstituted araliphatic of 7-15 carbons, substituted or unsubstituted aryl of 6-14 carbons, polyoxyalkylene of about 9-44 alkyleneoxide linkages or a radical of Formula II or III;

$R^2$ and $R^3$ may be concatenated to form a piperidine ring;

$R^4$ is selected from alkylene diradicals of 2-10 carbons;

$R^5$ is selected from hydrogen, alkyl of 1-4 carbons, methoxy, ethoxy, chloro, or carboxy;

$R^6$ is selected from methyl, ethyl, tert.-butyl, tert.-octyl, or 1-methyl-1-phenylethyl;

$R^7$ is selected from hydrogen, acetyl or benzoyl; and

$R^8$ is selected from hydrogen, methyl, acetyl, or benzoyl.

**4.** Oxamides as defined in claim 1 wherein:

R is selected from hydrogen, substituted or unsubstituted aliphatic of 1-8 carbons, substituted or unsubstituted alkoxy of 1-8 carbons, methoxycarbonyl, ethoxycarbonyl, carboxy, chloro or $S(=O)_2$-OH; $R^1$ may be hydrogen, substituted or unsubstituted aliphatic of 1-8 carbons, or substituted or unsubstituted araliphatic of 7-9 carbons;

$R^2$ is selected from hydrogen or lower alkyl of 1-6 carbons;

$R^3$ is selected from hydrogen, substituted or unsubstituted aliphatic of 1-18 carbons, substituted or

unsubstituted alicyclic of 6-10 carbons, substituted or unsubstituted araliphatic of 7-15 carbons, substituted or unsubstituted aryl of 6-14 carbons, polyoxyalkylene of about 9-44 alkyleneoxide linkages or a radical of Formula II wherein X is a direct bond or III;

$R^2$ and $R^3$ may be concatenated to form together with the nitrogen atom to which they are attached a substituted or unsubstituted piperidine, piperazine or morpholine ring.

**5.** Oxamides as defined in claim 4 wherein:

R is selected from hydrogen or chlorine;

$R^1$ is selected from alkyl of 1-8 carbons or araliphatic of 7-9 carbons and is para to the hydroxyl substituent on the phenyl ring;

$R^2$ is selected from hydrogen or lower alkyl of 1-6 carbons; and

$R^3$ is selected from hydrogen, substituted or unsubstituted aliphatic of 1-18 carbons, substituted or unsubstituted alicyclic of 6-10 carbons, substituted or unsubstituted araliphatic of 7-15 carbons, substituted or unsubstituted aryl of 6-14 carbons or polyoxyalkylene of about 9-44 alkyleneoxide linkages.

**6.** Oxamides as defined in claim 5 wherein:

R is hydrogen;

$R^1$ is methyl;

$R^2$ is hydrogen; and

$R^3$ is selected from hydrogen, substituted or unsubstituted aliphatic of 3-18 carbons, cyclohexyl, polyoxyalkylene of about 9-44 alkyleneoxide linkages or substituted or unsubstituted aryl of 6-14 carbons wherein the substituents for the aliphatic and aryl groups are selected from hydroxy, methoxy or ethoxy.

**7.** Oxamides as defined in claim 1 wherein:

R is selected from hydrogen, substituted or unsubstituted aliphatic of 1-8 carbons, substituted or unsubstituted alkoxy of 1-8 carbons, methoxycarbonyl, ethoxycarbonyl, carboxy, chloro or $S(=O)_2$-OH;

$R^1$ is selected from hydrogen, substituted or unsubstituted aliphatic of 1-8 carbons, or substituted or unsubstituted araliphatic of 7-9 carbons;

$R^2$ is selected from hydrogen or lower alkyl of 1-6 carbon; and

$R^3$ is selected from a radical of Formula II wherein X is is a direct bond or III.

**8.** Oxamides as defined in claim 7 wherein:

$R^3$ is a radical of Formula II where X is a direct bond;

R and $R^5$ are independently selected from hydrogen or chlorine;

$R^2$ is hydrogen; and

$R^1$ and $R^6$ are independently selected from substituted or unsubstituted aliphatic of 1-8 carbons or substituted or unsubstituted araliphatic of 7-9 carbons and are para to the hydroxyl substituents on the phenyl rings.

**9.** Oxamides as defined in claim 7 wherein:

$R^3$ is a radical of Formula III;

R is selected from hydrogen or chlorine;

$R^1$ is selected from substituted or unsubstituted aliphatic of 1-8 carbons or substituted or unsubstituted araliphatic of 7-9 carbons and is para to the hydroxyl substituent on the phenyl ring;

$R^2$ is selected from hydrogen or lower alkyl of 1-6 carbons; and

$R^8$ is selected from hydrogen, alkyl of 1-4 carbons, alkenyl of 3 or 4 carbons, benzyl, acetyl or benzoyl.

**10.** Oxamides as defined in claim 1 wherein:

$R^3$ is Formula II wherein X is

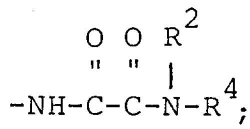

R is selected from hydrogen or chlorine;

$R^1$ is selected from substituted or unsubstituted aliphatic of 1-8 carbons or substituted or unsubstituted araliphatic of 7-9 carbons and is para to the hydroxyl substituent on the phenyl ring;

$R^2$ is selected from hydrogen or lower alkyl of 1-6 carbons; and

$R^4$ is selected from substituted or unsubstituted aliphatic diradicals of 1-20 carbons, substituted or unsubstituted aryl diradicals of 6-12 carbons, substituted or unsubstituted alicyclic diradicals of 5-12 carbons, substituted or unsubstituted araliphatic diradicals of 7-22 carbons, the aliphatic chains of which may optionally contain heteroatoms -O-, -S- and -N($R^7$)- with the proviso that multiple heteroatoms must be separated from each other and the diradical ends by at least two carbon atoms, or polyoxyalkylene diradicals of about 3-34 alkyleneoxide linkages.

**11.** Oxamides as defined in claim 10 wherein:

R is hydrogen;

$R^1$ is selected from methyl or tert.-octyl;

$R^2$ is hydrogen; and

$R^4$ is selected from substituted or unsubstituted aliphatic diradicals of 2-10 carbons, or polyoxyalkylene diradicals of about 3-34 alkyleneoxide linkages.

**12.** A process for preparing an oxamide as defined in claims 5, 8 or 9 wherein:

$R^2$ is hydrogen which comprises:

treating a N-[2-hydroxy-3-(2H-benzotriazol-2-yl)-5-(alkyl or aralkyl)benzyl]oxamate ester of Formula VIa

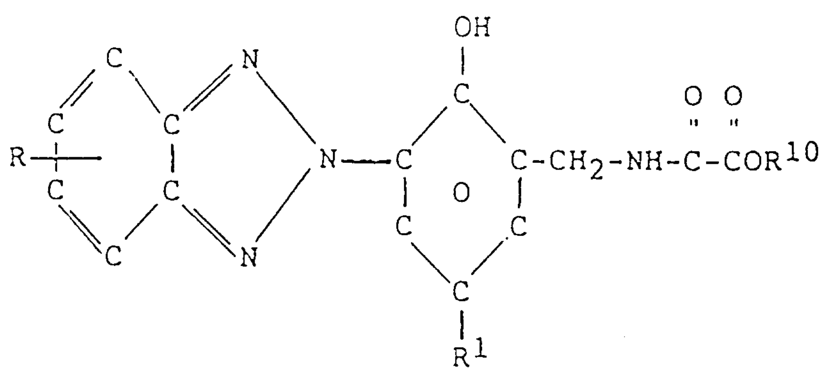

VIa

wherein R is selected from hydrogen or chlorine;

$R^1$ is selected from substituted or unsubstituted aliphatic of 1-8 carbons or substituted or unsubstituted araliphatic of 7-9 carbons and is para to the hydroxyl substituent on the phenyl ring; and

$R^{10}$ is lower alkyl of 1-4 carbons or phenyl;

with a primary amine of structure $R^3NH_2$;

wherein:

$R^3NH_2$ is selected from ammonia, substituted or unsubstituted aliphatic primary amines of 1-18 carbons, substituted or unsubstituted alicyclic primary amines of 6-10 carbons, substituted or unsubstituted araliphatic primary amines of 7-15 carbons, polyoxyalkylene primary amines of about 9-44 alkyleneoxide linkages, a 2-[2'-hydroxy-3'-aminomethyl-5-(alkyl or aralkyl)phenyl]-2H-benzotriazole of Formula IV:

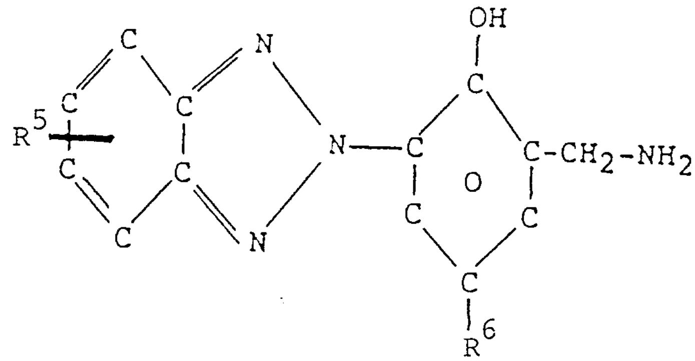

IV

wherein:

$R^5$ is selected from hydrogen or chlorine;

$R^6$ is selected from substituted or unsubstituted aliphatic of 1-8 carbons, or substituted or unsubstituted araliphatic of 7-9 carbons and is para to the hydroxyl substituent on the phenyl ring; or a 4-amino-substituted-2,2,6,6-tetramethylpiperidine of Formula X:

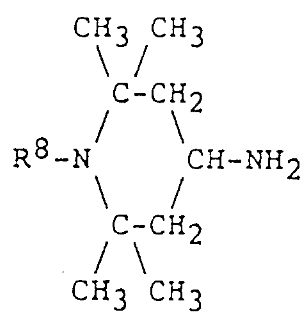

X

wherein:

$R^8$ is selected from hydrogen, alkyl of 1-4 carbons, alkenyl of 3 or 4 carbons, benzyl, acetyl or benzoyl;

in an inert polar solvent at or below the reflux temperature of the solvent.

**13.** A process as defined in claim 12 wherein:

R and $R^5$ are hydrogen;

$R^1$ and $R^6$ are methyl;

$R^3NH_2$ is selected from octylamine, decylamine, butylamine, allylamine, octadecylamine, 2-hydroxyethylamine, 3-dimethylaminopropylamine, 3-diethylaminopropylamine or 3-triethoxysilylpropylamine, polyoxyalkylene primary amines of about 9-44 alkyleneoxide linkages; 2-(2'-hydroxy-3'-aminomethyl-5'-methylphenyl)-2H-benzotriazole and 4-amino-2,2,6,6-tetramethylpiperidine;

$R^{10}$ is selected from methyl or ethyl;

the inert polar solvent is selected from methanol, ethanol, propanol or isopropanol; and

the amine is mixed with the compound of Formula VIA in approximately a 1:1 mole ratio.

**14.** A process for preparing an oxamide as defined in claim 10 wherein:

$R^2$ is hydrogen which comprises:

treating a N-[2-hydroxy-3-(2H-benzotriazol-2-yl)-5-(alkyl or aralkyl)benzyl]oxamate ester of Formula Va;

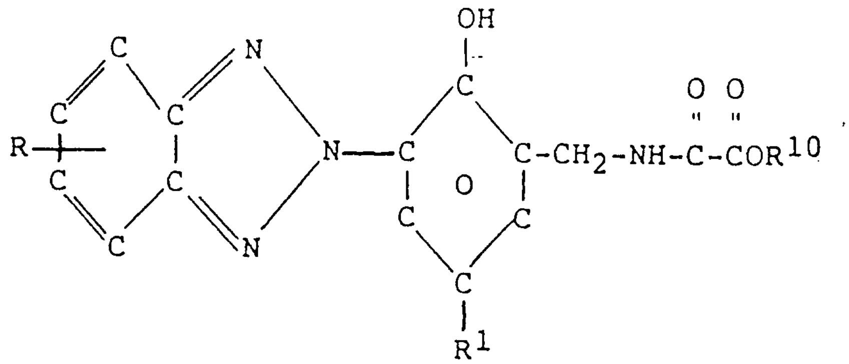

Va

wherein:

R is selected from hydrogen or chlorine;

$R^1$ is selected from substituted or unsubstituted aliphatic of 1-8 carbons or substituted or unsubstituted araliphatic of 7-9 carbons and is para to the hydroxyl substituent on the phenyl ring;

$R^{10}$ is selected from lower alkyl of 1-4 carbons or phenyl;

with a primary aliphatic, cycloaliphatic, araliphatic or polyoxyalkylene diamine of structure $H_2N$-$R^4$-$NH_2$, where $R^4$ is selected from substituted or unsubstituted aliphatic diradicals of 1-20 carbons, substituted or unsubstituted aryl diradicals of 6-12 carbons, substituted or unsubstituted alicyclic diradicals of 5-12 carbons, substituted or unsubstituted araliphatic diradicals of 7-22 carbons, the aliphatic chains of which may optionally contain -O-, -S- and -N($R^7$)- with the proviso that multiple heteroatoms must be separated from each other and the diradical ends by at least two carbon atoms, or polyoxyalkylene diradicals of about 3-34 alkyleneoxide linkages;

in a molar ratio of approximately 2:1 of the compound of Formula Va to the diamine in an inert polar solvent at or below the reflux temperature of the solvent.

**15.** A process as defined in claim 14 wherein:

R is hydrogen;

$R^1$ is selected from methyl or tert.-octyl;

$R^4$ is selected from a substituted or unsubstituted aliphatic diradicals of 2-10 carbons or polyoxyalkylene diradicals of about 3-34 alkyleneoxide linkages; and

the inert solvent is selected from methanol, ethanol, propanol or isopropanol.

**16.** A process for preparing an oxamide as defined in claim 8 which comprises:

treating a sulfuric acid solution of a 2-(2-hydroxyphenyl)-2H-benzotriazole of Formula VIII,

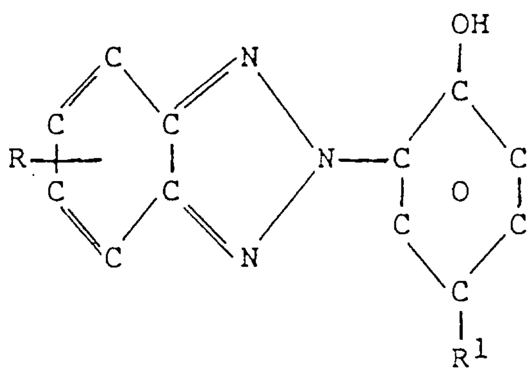

VIII

wherein:

R is selected from hydrogen or chlorine; and

$R^1$ is selected from substituted or unsubstituted aliphatic of 1-8 carbons or substituted or unsubstituted araliphatic of 7-9 carbons and is para to the hydroxyl substituent on the phenyl ring;

with N,N'-bis(hydroxymethyl)oxamide at -5 to 30 °C in approximately a 2:1 mole ratio of the compound of Formula VIII to N,N'-bis(hydroxymethyl)oxamide.

**17.** A process as defined in claim 16 wherein:

R and $R^5$ are hydrogen; and

$R^1$ and $R^6$ is selected from methyl or tert.-octyl; and the treatment is carried out by adding one molar part N,N'-bis(hydroxymethyl)oxamide to a solution of two molar parts 2-(2H-benzotriazol-2-yl)-4-methylphenol in 95 to 100% sulfuric acid while holding the temperature between -5 and 15 °C.

**18.** A process as defined in claim 17 wherein:

$R^1$ and $R^6$ are methyl.

**19.** A process as defined in claim 17 wherein:

$R^1$ and $R^6$ are tert.-octyl.

**20.** A process for stabilizing a polymer composition selected from synthetic polymers, natural polymers or a mixture of synthetic and natural polymers against light induced degradation which comprises mixing said polymer composition with a stabilizing effective amount of an oxamide as defined in claim 1 to provide a polymer composition-oxamide mixture, optionally containing one or more property enhancing additive selected from hindered amine light stabilizers, hindered phenols, other o-hydroxyphenyl-2H-benzotriazoles, 2-hydroxybenzophenones, phosphites, phosphonites or combinations thereof.

**21.** A process as defined in claim 20 wherein:

the polymer is a synthetic polymer and is selected from polyolefins, polyvinyl halides, polyvinylidene halides, polyacrylates, polymethacrylates, polystyrenics, rubber modified styrenics, polyphenylene ethers, polycarbonates, polyestercarbonates, polyamides, polyacetals, polyurethanes or acrylonitrile-butadiene-styrene terpolymers, wherein the oxamide is present in the polymer composition in a concentration of 0.01 to 5.0% by weight.

**22.** A process as defined in claim 20 wherein:

the polymer is selected from blends of polycarbonate and acrylonitrile-butadiene-styrene terpolymer; and the oxamide is N,N'-bis[2-hydroxy-3-(2H-benzotriazol-2-yl)-5-(tert.-octyl)benzyl]-oxamide.

**23.** A stabilized polymer composition comprising a polymer selected from the group consisting of natural polymers, synthetic polymers and mixtures thereof and a stabilizing effective amount of an oxamide as defined in claim 1.

European Patent Office

# EUROPEAN SEARCH REPORT

Application Number
EP 93 11 5443

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| Y | WO-A-90 09369 (PENNWALT CORPORATION)<br>* the whole document, particularly examples 3 and 16-18 *<br>--- | 1-23 | C07D249/20<br>C08K5/3475<br>C07D401/12<br>C07F7/18<br>C08K5/54 |
| D,Y | EP-A-0 191 582 (TAKEMOTO YUSHI KABUSHIKI KAISHA)<br>* the whole document *<br>--- | 1-23 | |
| Y | GB-A-2 196 966 (SANDOZ LTD)<br>* the whole document *<br>----- | 1-23 | |

TECHNICAL FIELDS SEARCHED (Int.Cl.5)

C07D
C07F
C08K

The present search report has been drawn up for all claims

| Place of search | Date of completion of the search | Examiner |
|---|---|---|
| THE HAGUE | 3 February 1994 | Allard, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document